(19) Europäisches Patentamt
European Patent Office
Office européen des brevets

(11) EP 3 929 192 A1

(12) EUROPEAN PATENT APPLICATION

(43) Date of publication:
**29.12.2021 Bulletin 2021/52**

(21) Application number: **20305691.6**

(22) Date of filing: **23.06.2020**

(51) Int Cl.:
***C07D 405/14*** *(2006.01)* ***A61P 25/16*** *(2006.01)*
***A61P 25/28*** *(2006.01)* ***A61K 31/513*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Université de Franche-Comté**
**25030 Besançon (FR)**

(72) Inventor: **ISMAILI, Lhassane**
**25000 Besançon (FR)**

(74) Representative: **Plasseraud IP**
**66, rue de la Chaussée d'Antin**
**75440 Paris Cedex 09 (FR)**

# (54) COMPOUNDS FOR TREATING NEURODEGENERATIVE DISEASES

(57) The present invention relates to compounds of formula (I) as well as pharmaceutically acceptable salts thereof, wherein the substituents are as those disclosed in the present disclosure, and their use as a medicament, in particular for the treatment of neurodegenerative diseases.

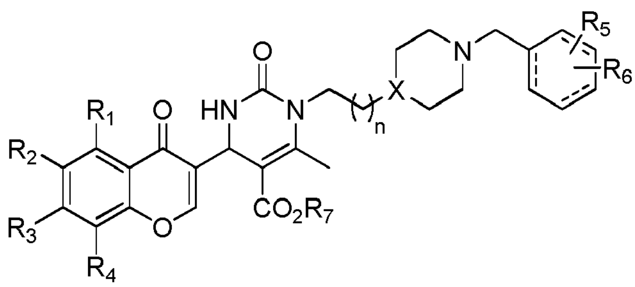

(I)

EP 3 929 192 A1

## Description

## BACKGROUND

In the central nervous system, memory and cognitive processes condition throughout our lives, our ability to learn and communicate as well as our psychomotor development. However, disturbances in memory and learning abilities can occur with age. Thus, with the increase in life expectancy worldwide, there is an alarming increase in the prevalence of neurodegenerative pathologies such as Alzheimer's disease (AD) in the elderly.

This disease leads to severe cognitive deficits such as disorientation, depression, and even inability to perform the tasks of daily living[1].

AD is characterized by a series of highly interconnected pathological processes that result, biologically and biochemically, in the accumulation and aggregation of an abnormal amount of extracellular deposits of amyloid-beta peptide (βA) that cause senile plaques and neurofibrillary degeneration, composed of hyperphosphorylated tau protein, leading to neuronal death[2]. For the extracellular deposits (βA), several strategies have been developed to avoid these plaques and stop the progression of the disease, for example, using compounds that may act on the release of the peptide βA[3,4] or its aggregation[5].

Concerning the deposits related to neurofibrillary degeneration resulting from hyperphosphorylation of the tau protein, the main therapeutic strategies target the oligomerization of the protein and the inhibition this hyperphosphorylation[6,7], in particular the inhibition the glycogen synthase kinase 3 (GSK-3β) which is the main kinase responsible for the phosphorylation of the Tau protein[8].

Besides, over the last few decades, a cholinergic approach has brought 4 drugs to market for the treatment of the disease: Tacrine, Galanthamine, Rivastigmine and Donepezil. They are capable of inhibiting acetycholinesterase (AChE) and butyrylcholinesterase (BuChE), allowing an increase in neurotransmission between synapses to compensate for the cholinergic deficit by preventing hydrolysis of the neurotransmitter[9].

Another strategy for AD therapy is the development of drugs such as Nivaldipine, currently in phase 3 clinical development, able to block calcium channels[10]. Indeed, the increase in the level of cytosolic calcium is involved in the physiopathology of AD, as it facilitates the formation of peptide βA by increasing the activity of calcium-induced beta secretase[11-15] and regulates certain kinases such as GSK-3β[16]. In addition, the entry of calcium through L-type calcium channels results in calcium overload and mitochondrial disruption leading to cell apoptosis activation[17].

The Inventors have discovered that derived compounds from ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate according to formula I, had an action on both calcium channel blockade, inhibition of cholinesterases (AChE and BuChE), GSK-3β, and monoamine oxidase enzymes (MAO) as well as inhibition of Tau aggregation and antioxidant activity.

In fact, oxidative stress also plays a role in the pathophysiology of AD[18]. The accumulation of reactive oxygen species (ROS) and reactive nitrogen species (RNS) leads to the oxidation of the main components of neurons (lipids, proteins and nucleic acids) and thus to significant damages to neurons[19,20]. This oxidative stress is caused by various underlying factors such as mitochondrial dysfunction[21,22], disturbance of the homeostasis of certain metals (Cu, Fe, Zn) and their involvement in peptide βA aggregation[18,23], neuroinflammation[24,25], or the production of hydrogen peroxide ($H_2O_2$) in the MAO-catalyzed deamination reaction of biogenic amines[26,27]. There is consensus that these etiological mechanisms all coexist at the same time, influencing each other at multiple levels[28].

Thus, the compounds according to the invention have the advantage of targeting several causes of AD.

## SUMMARY OF THE INVENTION

The present invention is directed to a compound of formula (I)

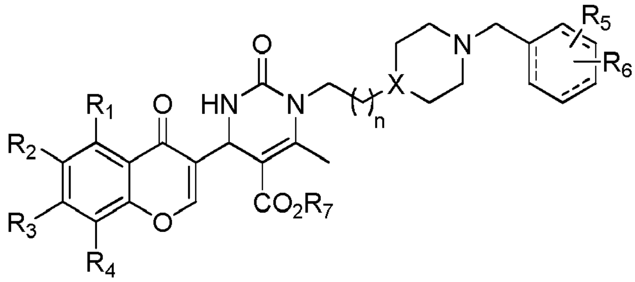

**(I)**

including any stereochemically isomeric form thereof,

wherein:

$R_1$, $R_2$, $R_3$ and $R_4$ independently of each other, are H, halogen, substituted or unsubstituted $C_1$-$C_6$ alkyl, $NO_2$, OH, O-methyl, CN, acyl, alkoxycarbonyl, SH or NR'R" with R' and R" being either or independently of each other hydrogen, alkyl, substituted or not aryl, or R' and R" forming together a cycloalkyl;

$R_5$ and $R_6$, independently of each other, are H, halogen, substituted or unsubstituted $C_1$-$C_6$alkyl, cycloalkyl, substituted or not aryl, $NO_2$, OH, CN, acyl, alkoxy, alkoxycarbonyl, SH;

$R_7$ is a substituted or unsubstituted $C_1$-$C_6$ alkyl or a substituted or unsubstituted aryl;

X is N or CH;

n is 0, 1 or 2;

the symbol $\overline{- - -}$ indicates a single or double bond;

or a pharmaceutically acceptable salt thereof.

The present invention is also directed to a pharmaceutical composition comprising a compound according to formula (I), or a pharmaceutically acceptable salt thereof, and a pharmaceutically acceptable carrier.

The present invention is further directed to a compound according to formula (I) or a pharmaceutically acceptable salt thereof, for use as a medicament, in particular for use in the treatment of neurodegenerative diseases.

## DETAILED DESCRIPTION

A compound according to the invention is inherently intended to comprise all stereochemically isomeric forms thereof. The term "stereochemically isomeric forms" as used hereinbefore or hereinafter defines all the possible stereoisomeric forms which the compounds of formula (I), may possess. Compounds of formula (I) can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates or mixtures of diastereoisomeric racemates. The optically active forms can be obtained for example by resolution of the racemates, by asymmetric synthesis or asymmetric chromatography (chromatography with a chiral adsorbents or eluant). The invention embraces all of these forms. Unless otherwise mentioned or indicated, the chemical designation of compounds denotes the mixture of all possible stereochemically isomeric forms. In particular, stereogenic centers may have the D (+) or L (-) configuration; the R- or S-configuration; substituents on bivalent cyclic (partially) saturated radicals may have either the cis- or trans-configuration. Compounds encompassing double bonds can have an E (entgegen) or Z (zusammen)-stereochemistry at said double bond. The terms D, (+), L, (-), cis, trans, R, S, E and Z are well known to a person skilled in the art.

According to a first aspect, the present invention concerns a compound of formula (I):

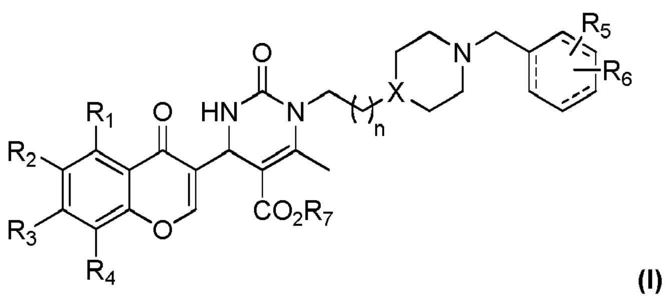

**(I)**

including any stereochemically isomeric form thereof,
wherein:

$R_1$, $R_2$, $R_3$ and $R_4$ independently of each other, are H, halogen, substituted or unsubstituted $C_1$-$C_6$ alkyl, $NO_2$, OH, O-methyl, CN, acyl, alkoxycarbonyl, SH or NR'R" with R' and R" being either or independently of each other hydrogen, alkyl, substituted or not aryl, or R' and R" forming together a cycloalkyl;

$R_5$ and $R_6$, independently of each other, are H, halogen, substituted or unsubstituted $C_1$-$C_6$ alkyl, cycloalkyl, substituted or not aryl, $NO_2$, OH, CN, acyl, alkoxy, alkoxycarbonyl, SH;

$R_7$ is a substituted or unsubstituted $C_1$-$C_6$ alkyl or a substituted or unsubstituted aryl;

X is N or CH;

n is 0, 1 or 2;

the symbol $\overline{- - -}$ indicates a single or double bond;

or a pharmaceutically acceptable salt thereof.

As used herein, the term "halogen" means Br, Cl, I and F, preferably Br or Cl.

The term "alkyl" as used herein denotes a substituted or unsubstituted chain, saturated, monovalent hydrocarbon residue containing 1 to 6 carbon atoms. "$C_1$-$C_6$ alkyl" as used herein refers to an alkyl composed of 1 to 6 carbons. Examples of alkyl groups include, but are not limited to, methyl, ethyl, propyl, isopropyl, butyl, sec-butyl, isobutyl, tert-butyl, pentyl and hexyl.

When substituted, there will generally be, for example, 1 to 4 substituents present on the alkyl. These substituents may optionally form a ring with the alkyl with which they are connected. Substituents may include, for example: carbon-containing groups such as alkyl, aryl, arylalkyl (e.g. substituted and unsubstituted phenyl, substituted and unsubstituted benzyl); halogen atoms and halogen-containing groups such as haloalkyl (e.g. trifluoromethyl); oxygen-containing groups such as alcohols (e.g. hydroxyl, hydroxyalkyl, aryl(hydroxyl)alkyl), ethers (e.g. alkoxy, aryloxy, alkoxyalkyl, aryloxyalkyl, more preferably, for example, methoxy and ethoxy), aldehydes (e.g. carboxaldehyde), ketones (e.g. alkylcarbonyl, alkylcarbonylalkyl, arylcarbonyl, arylalkylcarbonyl, arycarbonylalkyl), acids (e.g. carboxy, carboxyalkyl), acid derivatives such as esters (e.g. alkoxycarbonyl, alkoxycarbonylalkyl, alkylcarbonyloxy, alkylcarbonyloxyalkyl), amides (e.g. aminocarbonyl, mono- or di-alkylaminocarbonyl, aminocarbonylalkyl, mono- or di-alkylaminocarbonylalkyl, arylaminocarbonyl), carbamates (e.g. alkoxycarbonylamino, aryloxycarbonylamino, aminocarbonyloxy, mono- or di-alkylaminocarbonyloxy, arylminocarbonloxy) and ureas (e.g. mono- or di-alkylaminocarbonylamino or arylaminocarbonylamino); nitrogen-containing groups such as amines (e.g. amino, mono- or di-alkylamino, aminoalkyl, mono- or di-alkylaminoalkyl), azides, nitriles (e.g. cyano, cyanoalkyl), nitro; sulfur-containing groups such as thiols, thioethers, sulfoxides and sulfones (e.g. alkylthio, alkylsulfinyl, alkylsulfonyl, alkylthioalkyl, alkylsulfinylalkyl, alkylsulfonylalkyl, arylthio, arysulfinyl, arysulfonyl, arythioalkyl, arylsulfinylalkyl, arylsulfonylalkyl); and heterocyclic groups containing one or more heteroatoms, (e.g. thienyl, furanyl, pyrrolyl, imidazolyl, pyrazolyl, thiazolyl, isothiazolyl, oxazolyl, oxadiazolyl, thiadiazolyl, aziridinyl, azetidinyl, pyrrolidinyl, pyrrolinyl, imidazolidinyl, imidazolinyl, pyrazolidinyl, tetrahydrofuranyl, pyranyl, pyronyl, pyridyl, pyrazinyl, pyridazinyl, piperidyl, hexahydroazepinyl, piperazinyl, morpholinyl, thianaphthyl, benzofuranyl, isobenzofuranyl, indolyl, oxyindolyl, isoindolyl, indazolyl, indolinyl, 7-azaindolyl, benzopyranyl, coumarinyl, isocoumarinyl, quinolinyl, isoquinolinyl, naphthridinyl, cinnolinyl, quinazolinyl, pyridopyridyl, benzoxazinyl, quinoxalinyl, chromenyl, chromanyl, isochromanyl, phthalazinyl and carbolinyl).

As used herein, "substituted or not aryl" means that said aryl may be unsubstituted or substituted by one or more substituents selected from the group consisting of alkyl, halogen, haloalkyl, alkoxy, haloalkoxy, alkylamino, $NO_2$, OH, CN, acyl, alkoxycarbonyl, SH.

The term "alkoxy" as used herein means an -O-alkyl group, wherein alkyl is as defined above.

According to the present application, "pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

A "pharmaceutically acceptable salt" form of an active ingredient may also initially confer a desirable pharmacokinetic property on the active ingredient which were absent in the non-salt form, and may even positively affect the pharmacodynamics of the active ingredient with respect to its therapeutic activity in the body. The phrase "pharmaceutically acceptable salt" of a compound means a salt that is pharmaceutically acceptable and that possesses the desired pharmacological activity of the parent compound. Such salts include acid addition salts, formed with inorganic acids such as hydrochloric acid, hydrobromic acid, sulfuric acid, methanesulfonic acid, p-toluenesulfonic acid, carbonic acid, photonic acid, acetic acid, oxalic acid, succinic acid, tartaric acid, mandelic acid, fumaric acid, maleic acid, lactic acid, citric acid, glutamic acid, saccharic acid, monomethylimbic acid, 5-oxoproline acid, hexane-1-sulphonic acid, nicotinic acid, 2, 3 or 4-aminobenzoic acid, 2,4,6-trimethylbenzoic acid, lipoic acid, aspartic acid, hydrochloric acid. Other examples are mono- or bis-oxalates salt.

In a particular embodiment of Formula (I), X is N. In a particular embodiment of Formula (I), X is CH. In a particular embodiment of Formula (I), $R_5$ and $R_6$ are H. In a particular embodiment of Formula (I), $R_7$ is a $C_1$-$C_6$ alkyl

such as an ethyl. In a particular embodiment of Formula (I), $R_1$, $R_2$, $R_3$ and $R_4$ independently of each other, are H, halogen or unsubstituted C1-$C_6$ alkyl.

In a particular embodiment of Formula (I), $R_5$ and $R_6$ are H, and $R_7$ is a $C_1$-$C_6$ alkyl such as an ethyl.

In a particular embodiment of Formula (I), X is N and $R_5$ and $R_6$ are H. In a particular embodiment of Formula (I), X is N and $R_7$ is a $C_1$-$C_6$ alkyl. In a particular embodiment of Formula (I), X is N and $R_7$ is an ethyl. The compound is then of the following formula (II):

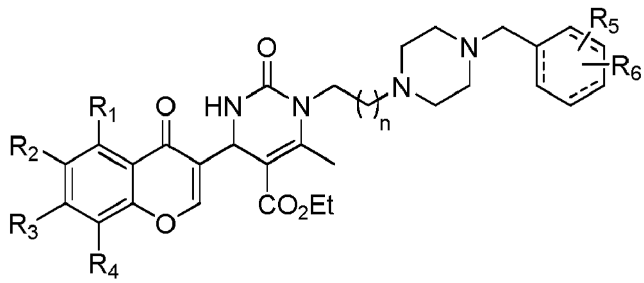

**(II)**

In a particular embodiment of Formula (I), X is N, $R_5$ and $R_6$ are H, and $R_7$ is a $C_1$-$C_6$ alkyl.

In a more particular embodiment of Formula (I), X is N, $R_5$ and $R_6$ are H, and $R_7$ is an ethyl. The compound is then of the following formula (III):

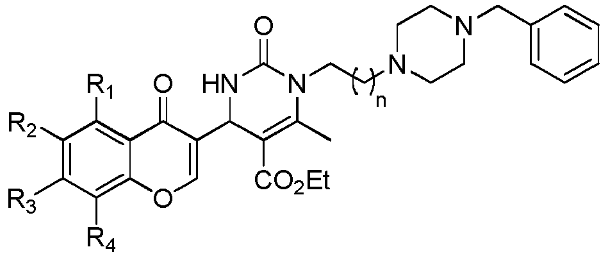

**(III)**

In a particular embodiment, the compound is a compound of the following formula (IV):

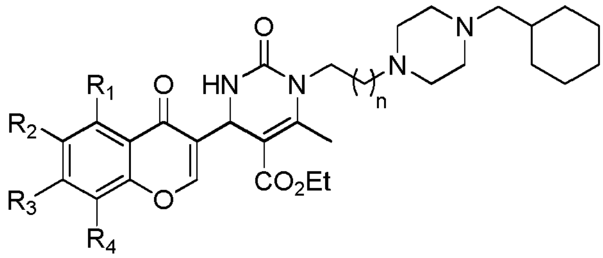

**(IV)**

In a particular embodiment of Formula (I), X is CH and $R_5$ and $R_6$ are H. In a particular embodiment of Formula (I), X is CH and $R_7$ is a $C_1$-$C_6$ alkyl. In a more particular embodiment of Formula (I), X is CH and $R_7$ is an ethyl. The compound is then of the following formula (V):

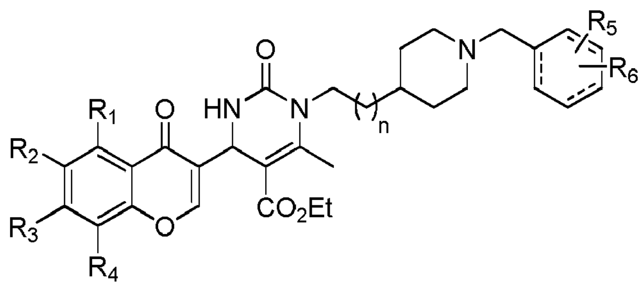

**(V)**

In a particular embodiment of Formula (I), X is CH, $R_5$ and $R_6$ are H, and $R_7$ is a $C_1$-$C_6$ alkyl. In a more particular embodiment of Formula (I), X is CH, $R_5$ and $R_6$ are H, and $R_7$ is an ethyl. The compound is then of the following formula (VII):

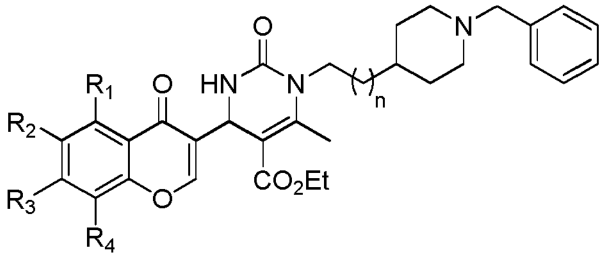

**(VII)**

In a particular embodiment, the compound is a compound of the following formula (VI):

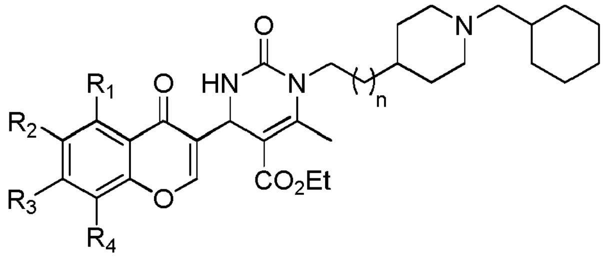

**(VI)**

In particular, the present invention relates to the particular embodiments of Formula (I) previously described, wherein $R_1$, $R_2$, $R_3$ and $R_4$ independently of each other, are H, halogen or unsubstituted $C_1$-$C_6$ alkyl.

In a particular embodiment, the compound of the present invention is a compound according to any of the particular embodiments of formula (I) previously described, or according to formula (VII) wherein $R_1$ is H.

In a particular embodiment, the compound of the present invention is a compound according to any of the particular embodiments of formula (I) previously described, or according to formula (VII) wherein $R_2$ is H, a halogen, in particular Br or Cl, or a $C_1$-$C_6$ alkyl such as isopropyl.

In a particular embodiment, the compound of the present invention is a compound according to any of the particular embodiments of formula (I) previously described, or according to formula (VII) wherein $R_3$ is H or a $C_1$-$C_6$ alkyl such as methyl or ethyl.

In a particular embodiment, the compound of the present invention is a compound according to any of the particular embodiments of formula (I) previously described, or according to formula (VII) wherein $R_4$ is H or a halogen such as Cl.

In a particular embodiment, the compound is a compound according to any of the particular embodiments of formula (I) previously described, or according to formula (VII) wherein $R_1$ is H, $R_2$ is a halogen, in particular Cl or Br.

In a particular embodiment, the compound is a compound according to any of the particular embodiments of formula (I) previously described, or according to formula (VII) wherein $R_1$ and $R_3$ are H, $R_2$ is a halogen, in particular Cl or Br.

In a particular embodiment, the compound is a compound according to any of the particular embodiments of formula (I) previously described, or according to formula (VII) wherein $R_1$ is H and $R_2$ is a $C_1$-$C_6$ alkyl, in particular ethyl or isopropyl.

In a particular embodiment, the compound is a compound according to any of the particular embodiments of formula (I) previously described, or according to formula (VII) wherein $R_2$ is a $C_1$-$C_6$ alkyl, in particular ethyl or isopropyl, and $R_1$, $R_3$, $R_4$ are H.

In a particular embodiment, the compound is a compound according to any of the particular embodiments of formula (I) previously described, or according to formula (VII) wherein $R_2$ and $R_3$ are a $C_1$-$C_6$ alkyl, in particular methyl or ethyl.

In a particular embodiment, the compound is a compound according to any of the particular embodiments of formula (I) previously described, or according to formula (VII) wherein $R_1$ is H, $R_2$ is a halogen, in particular Cl or Br, and wherein $R_3$ and $R_4$ independently of each other are H, a $C_1$-$C_6$ alkyl, in particular methyl, or a halogen, in particular Cl.

In a particular embodiment, the compound is a compound according to any of the particular embodiments of formula (I) previously described, or according to formula (VII) wherein $R_1$ is H, $R_2$ is a halogen, in particular Cl or Br, and wherein $R_3$ is a $C_1$-$C_6$ alkyl, in particular methyl and $R_4$ is H, or wherein $R_3$ is H and $R_4$ is a halogen, in particular Cl.

In a particular embodiment, the compound is a compound according to any of the particular embodiments of formula (I) previously described, or according to formula (VII) wherein $R_1$, $R_3$ and $R_4$ are H, and $R_2$ is H, a halogen, in particular Br, or a $C_1$-$C_6$ alkyl, in particular ethyl or isopropyl.

In a particular embodiment, the compound is a compound according to any of the particular embodiments of formula (I) previously described, or according to formula (VII) wherein $R_1$, $R_3$ and $R_4$ are H, and $R_2$ is a $C_1$-$C_6$ alkyl, in particular ethyl or isopropyl.

In a particular embodiment, the compound is a compound according to any of the particular embodiments of formula (I) previously described, or according to formula (VII) wherein $R_1$ and $R_4$ are H, $R_2$ is a halogen, in particular Cl, and $R_3$ is a $C_1$-$C_6$ alkyl, in particular methyl.

In a particular embodiment, the compound is a compound according to any of the particular embodiments of formula (I) previously described, or according to formula (VII) wherein $R_2$ and $R_4$ are a halogen, in particular Cl.

In a particular embodiment, the compound is a compound according to any of the particular embodiments of formula (I) previously described, or according to formula (VII) wherein $R_1$ and $R_3$ are H, $R_2$ and $R_4$ are a halogen, in particular Cl.

In a particular embodiment, the compound is a compound according to any of the particular embodiments of formula (I) previously described, or according to formula (VII) wherein $R_1$ and $R_4$ are H, $R_2$ and $R_3$ are $C_1$-$C_6$ alkyl, in particular $R_2$ is ethyl and $R_3$ is methyl.

In a particular embodiment, the compound is a compound according to any of the particular embodiments of formula (I) previously described, or according to formula (VII) wherein $R_1$, $R_2$ and $R_4$ are H, and $R_3$ is a $C_1$-$C_6$ alkyl, in particular an ethyl.

In a particular embodiment, the compound is a compound according to any of the particular embodiments of formula (I) previously described, or according to formula (VII) wherein $R_1$ and $R_4$ are H, $R_2$ is a halogen, in particular Cl, and $R_3$ is a $C_1$-$C_6$ alkyl, in particular a methyl.

In a particular embodiment, the compound is a compound according to any of the particular embodiments of formula (I) previously described, or according to formula (VII) wherein $R_1$ and $R_4$ are H, $R_2$ is H or a halogen such as Br, and $R_3$ is H or a $C_1$-$C_6$ alkyl such as an ethyl.

In a particular embodiment, the compound is a compound according to any of the particular embodiments of formula (I) previously described, or according to formula (VII) wherein $R_1$ is H, $R_2$ is a halogen such as Cl, $R_3$ is H or a $C_1$-$C_6$ alkyl such as a methyl, and $R_4$ is H or a halogen such as Cl.

In a particular embodiment, the compound is a compound according to any of the particular embodiments of formula (I) previously described, or according to formula (VII) wherein $R_1$, $R_2$, $R_3$ and $R_4$ are H.

In a particular embodiment, the compound is a compound according to any of the particular embodiments of formula (I) previously described, or according to formula (I) or formula (VII) wherein said compound is chosen in the group consisting of:

BIGI 3a

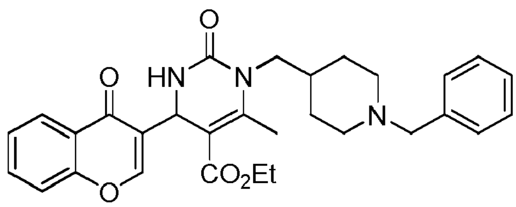

BIGI 3b

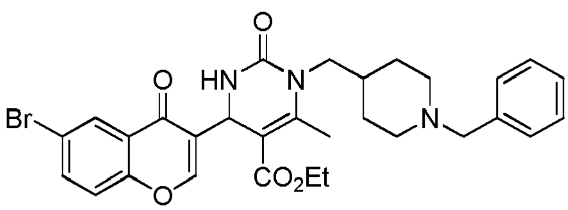

BIGI 3c

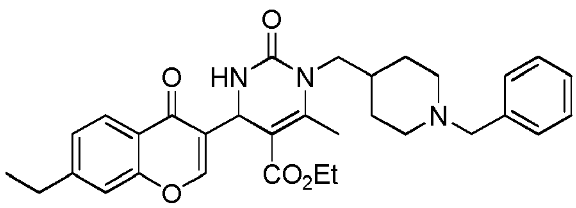

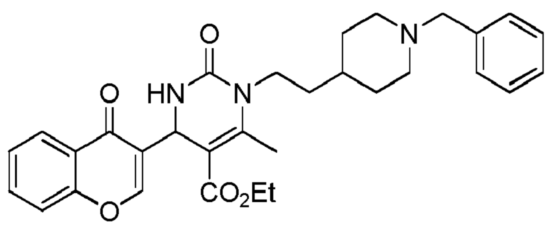

BIGI 3d

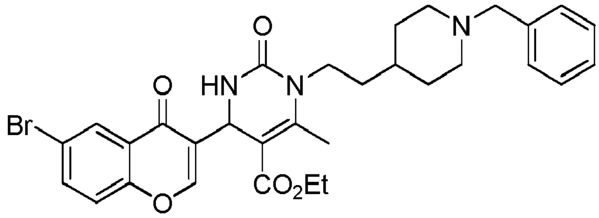

BIGI 3e

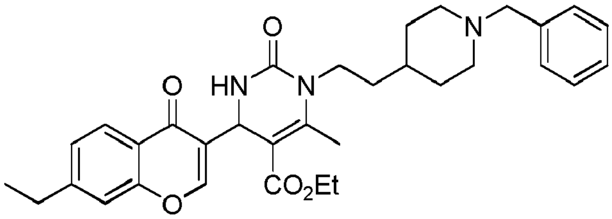

BIGI 3f

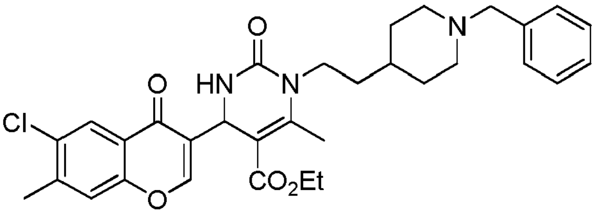

BIGI 3g

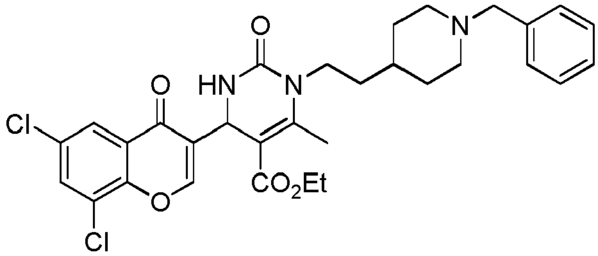

BIGI 3h

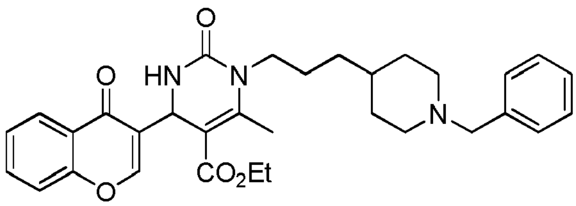

BIGI 3i

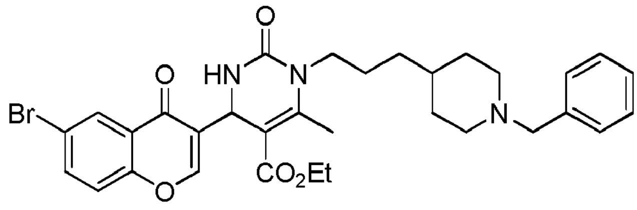

BIGI 3j

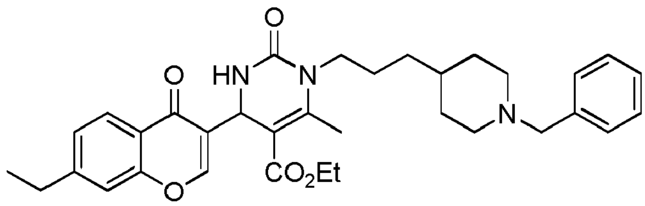

BIGI 3k

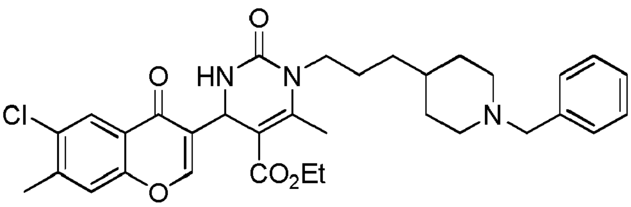

BIGI 3l

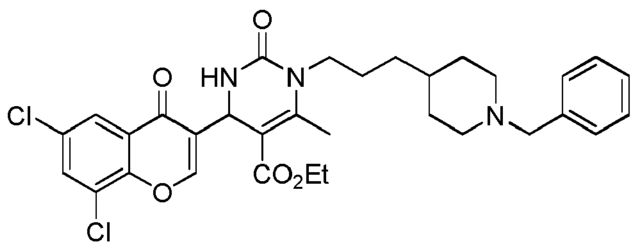

BIGI 3m

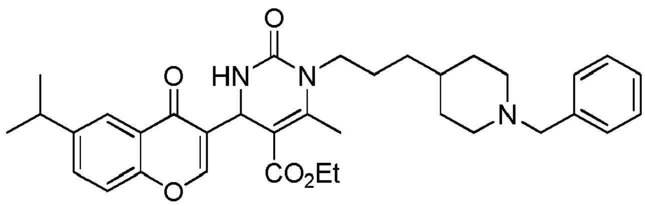

BIGI 3n

The present application also relates to a process for manufacturing compound of formula (VII) wherein a Biginelli reaction is performed between ethyl acetoacetate, an urea of formula (2a), (2b) or (2c),

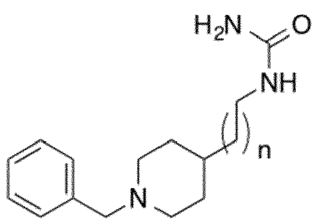

**2a n=0,**
**2b n=1,**
**2c n=2,**

and a 3-formyl chromone of formula (VIII)

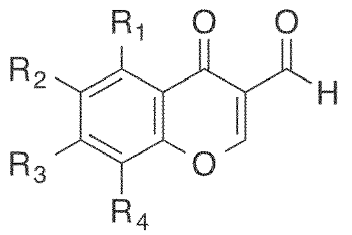

**(VIII)**

wherein $R_1$, $R_2$, $R_3$ and $R_4$ independently of each other, are H, halogen, substituted or unsubstituted $C_1$-$C_6$ alkyl, $NO_2$, OH, O-methyl, CN, acyl, alkoxycarbonyl, SH or NR'R" with R' and R" being either or independently of each other hydrogen, alkyl, substituted or not aryl, or R' and R" forming together a cycloalkyl.

In particular, said Biginelli reaction is performed with sodium bisulfate ($NaHSO_4$) as catalyst, and more particularly in presence of acetic acid.

In a particular embodiment, the process for manufacturing compound of formula (VII) comprises:

- a first reaction step between a 3-formyl chromone of formula (VIII) and an urea of formula (2a), (2b) or (2c), followed by

- a second reaction step wherein ethyl acetoacetate is added, in particular with sodium bisulfate (NaHSO4).

In particular, the first reaction step lasts 2h. Typically, the second reaction step lasts from 48h to 60h at room temperature (TA) followed by 4h under refluxing.

The present invention is also directed to a pharmaceutical composition comprising a compound as described above or a pharmaceutically acceptable salt thereof, and a pharmaceutically acceptable carrier.

The term "carrier" refers to a compound that is useful in the preparation of a pharmaceutical composition, generally safe, non-toxic and neither biologically nor otherwise undesirable, and includes excipients that are acceptable for human pharmaceutical uses. It encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body.

Useful pharmaceutical carriers for the preparation of the compositions hereof, can be solids, liquids or gases. The carrier can be selected from the various oils including those of petroleum, animal, vegetable or synthetic origin, e.g., peanut oil, soybean oil, mineral oil, sesame oil, and the like. Water, saline, aqueous dextrose, and glycols are representative liquid carriers, particularly (when isotonic with the blood) for injectable solutions. For example, formulations for intravenous administration comprise sterile aqueous solutions of the active ingredient(s) which are prepared by dissolving solid active ingredient(s) in water to produce an aqueous solution, and rendering the solution sterile.

Suitable pharmaceutical carriers include starch, cellulose, talc, glucose, lactose, talc, tragacanth, gelatin, pectin, dextrin, malt, rice, flour, chalk, silica, magnesium stearate, sodium stearate, glycerol monostearate, sodium chloride, methylcellulose, sodium carboxymethylcellulose, dried skim milk, glycerol, propylene glycol, water, ethanol, and the like. The pharmaceutical composition may be subjected to conventional pharmaceutical additives such as preservatives, stabilizing agents, wetting or emulsifying agents, salts for adjusting osmotic pressure, buffers, colorants, flavors, artificial and natural sweeteners, dispersants, thickeners, solubilizing agents and the like.

The compounds or compositions of the invention can thus be administered, for example, ocularly, orally (e.g., buccal cavity), sublingually, parenterally (e.g., intramuscularly, intravenously, or subcutaneously), rectally (e.g., by suppositories or washings), transdermally (e.g., skin electroporation) or by inhalation (e.g., by aerosol).

The composition of the invention may have a form of solid (e. g. include powders, tablets, coated tablets, dragees, pills, capsules, cachets, suppositories, and dispersible granules), liquid (e. g. solutions, emulsions, syrups, elixirs, or suspensions), gas or aerosol.

The present invention is further directed to a compound as described above or a pharmaceutically acceptable salt thereof, or a pharmaceutical composition comprising said compound or a pharmaceutically acceptable salt thereof as described above, for use as a medicament.

In particular, it relates to a compound as described above or a pharmaceutically acceptable salt thereof, or a pharmaceutical composition comprising said compound or a pharmaceutically acceptable salt thereof as described above, for use in the treatment of a neurodegenerative disease, a central nervous system disease, narcolepsy or sleep disorder.

In particular, the neurodegenerative disease is selected in the group consisting of: Alzheimer's disease, Parkinson's disease, amyotrophic lateral sclerosis.

The present invention also concerns the use of a compound as described above or a pharmaceutically acceptable salt thereof, in the manufacture of a medicament for treating a neurodegenerative disease, a central nervous system disease, narcolepsy or sleep disorder.

According to the present disclosure, the term "treating", with regard to a subject, refers to improving at least one symptom of the subject's disorder. Treating can be curing, improving, or at least partially ameliorating the disorder.

The present invention is further directed to a method for the treatment of a neurodegenerative disease, a central nervous system disease, narcolepsy or sleep disorder, comprising the step of administering to a patient in need thereof a therapeutically effective amount of a compound as described above or a pharmaceutically acceptable salt thereof, or a pharmaceutical composition comprising said compound or a pharmaceutically acceptable salt thereof as described above.

The term "therapeutically effective amount" as used herein means an amount required to reduce symptoms of the disease in an individual. The dose will be adjusted to the individual requirements in each particular case. That dosage can vary within wide limits depending upon numerous factors such as the severity of the disease to be treated, the age and general health condition of the patient, other medicaments with which the patient is being treated, the route and form of administration and the preferences and experience of the medical practitioner involved.

## FIGURES

**Figure 1:** Scheme of the synthesis of the derivate compounds of ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate of Formula (VII), in particular compounds BIGI-3a-3n.

**Figure 2:** Steady-state inhibition of AChE hydrolysis of substrate acetylthiocholine by compound BIGI-3e at different concentrations. Lineweaver-Burk reciprocal plots of initial velocity at increasing substrate concentrations (0.156 - 1.250 mM) are presented. Lines were derived from a linear regression of the data points.

**Figure 3:** Steady-state inhibition of AChE hydrolysis of substrate acetylthiocholine by compound BIGI-3h at different concentrations. Lineweaver-Burk reciprocal plots of initial velocity at increasing substrate concentrations (0.156 - 1.250 mM) are presented. Lines were derived from a linear regression of the data points.

**Figure 4:** Inhibition of Tau(306-336) peptide aggregation by compound BIGI-3h (noted BIGI21) and its isomers ISO1 (+) and ISO2 (-).

**Figure 5:** Effect of combined administration of scopolamine/dimethyl sulfoxide (scop/DMSO), and donepezil or compound BIGI-3h (noted bigi), on recognition index (RI). The animals are distributed into 5 experimental groups (I-V): control groups (I) mice administered 3.6% DMSO in saline buffer, labelled as "control vehicle"; experimental groups (II-V) consisted of (II) mice administered scopolamine (0,5 mg/kg) dissolved in 3.6% DMSO in saline buffer, labelled as "scop/DMSO," (III) mice co-administered scopolamine (0,5 mg/kg) plus Donepezil (1 mg/kg), labelled as "Scopo/donep," (IV) and (V) mice co-administrated scopolamine (0,5 mg/kg) plus BIGI-3h (bigi) at 1mg/Kg and 10mg/Kg labelled respectively "Scopo/bigi 1" "Scopo/bigi 10". Significant differences between scopo/DMSO and scopo/Bigi (10mg/kg) groups (**$p < 0.01$).

## MATERIAL & METHODS

### 1. Synthesis of the derivate compounds

### 1.1. Materials

All reagents were purchased from Sigma Aldrich or TCI. 1 H and $^{13}C$ NMR spectra were recorded on a Bruker spectrometer; operating at (1H NMR at 400 MHz, $^{13}C$ NMR at 100 MHz), in solution in dimethylsulfoxide (DMSO-$d_6$) at 20 °C; chemical shift values are given in $\delta$ (ppm) relatively to TMS as internal reference. Coupling constants are given in Hz. The following abbreviations were used: s = singlet, d = doublet, t = triplet, q = quartet, m = multiplet. High resolution mass spectra were obtained at Centre Commun de Spectrométrie de Masse, Lyon, France on a Bruker micrOTOF-Q II spectrometer (Bruker Daltonics) in positive ESI-TOF (electrospray ionization-time of flight). The TFAHs were found to be $\geq$ 95% pure by HPLC analysis using a Hitachi Lachrom Elite series instrument equipped with a L2400 Lachrom Elite DAD detector and a Uptisphere ODB column (4.6 mm x 100 mm, Ø= 3 $\mu$m). Peaks were detected at 210 nm and the system was operated at 25 °C with a flow rate of 2 mL/min. The mobile phase was an isocratic mixture of acetonitrile

and water (1:1, v/v) containing 0.1% (w/v) monopotassium phosphate.

### 1.2. General scheme of the synthesis

The derivate compounds of ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate (BIGI) has been carried out by a straightforward two-step synthesis with good overall yields, as depicted on Figure 1. The general scheme of the synthesis comprises a first step of urea synthesis, and a second step of a Biginelli reaction between said urea, ethyl acetoacetate, and different commercial 3-formyl chromones, in acetic acid with sodium bisulfate as catalyst.

### 1.3. Synthesis of ureas 2a-c[29]

To a solution of the corresponding 4-amino-benzylpiperidine 1a, 1b or 1c (1 g) in dichloromethane (10 mL) at 0°C was added a 20 mL solution of benzoyl isocyanate (1.2 eq.) in dichloromethane. The reaction was stirred for 1 hour at room temperature. The solvent was evaporated and the crude oil was recrystallized in diethyl ether to give a white solid. Yield: 72%. The product was reacted without further purification.

The corresponding benzoyl compound was hydrolyzed 48 hours at room temperature in a 1:1 solution of methanol in 1 M aqueous NaOH (30 mL). The solvents were concentrated under vacuum and extracted 3 times with ethyl acetate. The combined organic layers were washed with brine, dried over $Na_2SO_4$ and evaporated. The obtained solid was washed with diethyl ether to give a white solid. Yield: 93%.

### 1.4. Synthesis of compounds BIGI-3a-n by Biginelli reaction with ureas 2a-c

To a solution of 150 mg of the corresponding commercially chromones in 10 mL of acetic acid was added 1.2 equivalent of the corresponding urea 2a-c. The reaction was stirred for 2h. 1.25 equivalent of ethyl acetoacetate and 1 equivalent of sodium bisulfate were added. The reaction was stirred for 60 hours at room temperature and then refluxed 4h. The catalyst was filtered off and the solvent was evaporated under vacuum. The crude oil was purified by flash chromatography eluted with dichloromethane/MeOH/$NH_{3(aq)}$ 96:4:0.4 to give the pure product. Yield 45-78%.

The compounds BIGI-3a-n have been characterized by NMR.

## 2. Biological activities

### 2.1. Calcium channel antagonist assay

The cytosolic $Ca^{2+}$ has been measured in SH-SY5Y cells. SH-SY5Y cells were maintained similarly to what was previously described[30]. Free cytosolic $Ca^{2+}$ was measured using the fluorescent $Ca^{2+}$ indicator Fluo-4/AM (Fluo-4 acetomethoxyester). SH-SY5Y cells were seeded onto 96-well black plates at a density of $10^5$ cells per well, achieving confluence after 48 h. Cells were washed with Krebs-Hepes solution (KH, in mM: 144 NaCl, 5.9 KCl, 1.2 $MgCl_2$, 2 $CaCl_2$, 11 D-glucose, 10 HEPES, pH 7.4). Cells were loaded with 10 μM Fluo-4/AM and 0.2% pluronic acid in KH and incubated for 45 min at 37 °C in the dark. Then, cells were washed twice with KH to remove the excess of probe. Tested compounds were incubated 10 min before $K^+$ 70 mM was applied to evoke the increment of cytosolic $Ca^{2+}$. To normalize Fluo-4 signals, Triton X-100 (5%) and 1 M $MnCl_2$ were applied to register both maximal and minimal fluorescence, respectively. The experiments were analyzed at excitation and emission wavelengths of 485 and 520 nm, respectively, and fluorescence was measured in a fluorescence microplate reader (FLUOstar Optima, BMG, Germany). Data were calculated as a percentage of $F_{max}$ - $F_{min}$.

### 2.2. Cholinesterase inhibition in vitro assay

The AChE and BuChE inhibitory activity of the tested compounds was determined using a modified Ellman's method[31], [32].Human red-blood cell acetylcholinesterase (*h*AChE; EC 3.1.1.7), and human recombinant butyrylcholinesterase (*h*BuChE; EC 3.1.1.8), 5,5'-dithiobis(2-nitrobenzoic acid) (Ellman's reagent, DTNB), phosphate buffer (PB, pH 7.4), acetylthiocholine (ATC), and butyrylthiocholine (BTC), were purchased from Sigma-Aldrich (Prague, Czech Republic). Nunc polystyrene 96-well microplates with flat-bottom shape (ThermoFisher Scientific, USA) were used for the measurement and all the assay were carried out in 0.1 M $KH_2PO_4$/$K_2HPO_4$ buffer, pH 7.4. Enzyme solution was prepared at activity 2.0 units/mL in 2 mL aliquots. The assay mixture (100 μL) consisted of 40 μL of 0.1 M phosphate buffer (pH 7.4), 20 μL of 0.01 M DTNB, 10 μL of enzyme, and 20 μL of 0.01 M substrate (ATC iodide solution). Tested compounds were dissolved first in methanol (BIGI 23 in DMSO) and then further diluted by assay buffer to the final concentration. The highest concentration tested was 31.6 μM, which contained 0,5% of co-solvent (methanol or DMSO)

in the assay mixture. Such level of co-solvents was proved to have no effect on the activity of the enzyme. Enzyme with the inhibitor was preincubated for 5 min in the assay mixture and then the reaction was started by addition of 20 μL of substrate (ATC for AChE, BTC for BuChE). The enzyme activity was determined by measuring the increase in absorbance at 412 nm at 37 °C at 2 min intervals using a Multi-mode microplate reader Synergy 2 (Vermont, USA). Each concentration was assayed in triplicate. The obtained data were used to compute the percentage inhibition (/; Equation 1):

$$I = 1 - \frac{\Delta A_i}{\Delta A_0} \quad [\%] \qquad \text{(Eq. 1)}$$

$\Delta A_i$ indicates the absorbance change provided by cholinesterase exposed to AChE inhibitors and $\Delta A_0$ indicates absorbance change caused by intact cholinesterase (phosphate buffer was used instead of AChE inhibitor solution). The inhibition potency of the tested compounds was expressed as $IC_{50}$ value (the concentration of inhibitor which causes 50% cholinesterase inhibition). Calculations were performed using software Microsoft Excel (Redmont, WA, USA) and GraphPad Prism version 5.02 for Windows, GraphPad Software, San Diego, CA, USA.

**2.3. Oxygen Radical Absorbance Capacity (ORAC) Assay**

The radical scavenging capacity of the TFAHs was determined by the ORAC-FL method using fluorescein as a fluorescent probe[33,34]. (±)-6-Hydroxy-2,5,7,8-tetramethylchromane-2-carboxylic acid (Trolox), fluorescein (FL) and 2,2'-azobis(amidinopropane) dihydrochloride (AAPH) were purchased from Sigma-Aldrich. A Varioskan Flash plate reader with built-in injectors (Thermo Scientific) was used. The reaction was carried out at 37 °C in 75 mM phosphate buffer (pH= 7.4), and the final volume reaction mixture was 200 μL. The tested compounds and Trolox standard were dissolved in DMSO to 10 mM and further diluted in 75 mM phosphate buffer (pH= 7.4). The final concentrations were 0.1-1 μM for the tested compounds and 1-8 μM for Trolox standard. The blank was composed of 120 μL of FL, 60 μL of AAPH and 20 μL of phosphate buffer (pH=7.4) and was added in each assay. In a black 96-well microplate (Nunc), antioxidant (20 μL) and fluorescein (FL, 120 μL, final concentration of 70 nM were incubated for 15 min at 37 °C. Then, 2,2'-azobis(amidinopropane) dihydrochloride (AAPH, 60 μL, final concentration of 40 mM) solution was added rapidly using the built-in injector. The fluorescence was measured every minute for 60 min at 485 nm (excitation wavelength) and 535 nm (emission wavelength). The microplate was automatically shaken prior to each reading. All the reactions were made in triplicate and at least three different assays were performed for each sample. Antioxidant curves (fluorescence versus time) were first normalized to the curve of the blank (without antioxidant) and then, the area under the fluorescence decay curve (AUC) was calculated as: AUC=1+ sum($fi/f_0$), Where $f_0$ is the initial fluorescence reading at 0 min and *fi* is the fluorescence value at time *i*.

The net AUC corresponding to a sample was calculated as follows:

$$\text{Net AUC} = \text{AUC}_{\text{antioxidant}} - \text{AUC}_{\text{blank.}}$$

Regression equations were calculated by plotting the net AUC against the antioxidant concentration. The ORAC value was obtained by dividing the slope of the latter curve between the slope of the Trolox curve obtained in the same assay. Final ORAC values were expressed as μmol of Trolox equivalent/μmol of TFAH.

**2.4. Inhibition of $A\beta_{1-42}$ self-aggregation**

1,1,1,3,3,3-Hexafluoro-2-propanol (HFIP) pre-treated $A\beta_{1-42}$ samples (Bachem AG, Switzerland) were resolubilized with a MeCN/$Na_2CO_3$/NaOH (48.4/48.4/3.2) mixture to have a stable stock solution ([$A\beta_{1-42}$]=500 μM)[35]. Experiments were performed by incubating the peptide in 10 mM phosphate buffer (pH=8.0) containing 10 mM NaCl, at 30 °C for 24 h (final Aβ concentration=50 μM with and without inhibitor (50 μM). The inhibitor was dissolved in methanol and diluted in the assay buffer. Blanks containing inhibitor and ThT were also prepared and evaluated to account for quenching and fluorescence properties. To quantify amyloid fibril formation, the ThT fluorescence method was used[36], [37]. After incubation, samples were diluted to a final volume of 2.0 mL with 50 μM glycine-NaOH buffer (pH= 8.5) containing 1.5 μM ThT. A 300-seconds-time scan of fluorescence intensity was carried out ($\lambda_{exc}$=446 nm; $\lambda_{em}$=490 nm), and values at plateau were averaged after subtracting the background fluorescence of 1.5 μM ThT solution. The fluorescence intensities were compared and the % inhibition was calculated.

### 2.5. Prediction of blood-brain barrier penetration

To predict passive blood-brain penetration of novel compounds modification of the parallel artificial membrane permeation (PAMPA) assay has been used based on reported protocol[38, 39]. The filter membrane of the donor plate was coated with PBL (Polar Brain Lipid, Avanti, USA) in dodecane (4 μl of 20 mg/ml PBL in dodecane) and the acceptor well was filled with 300 μl of PBS pH 7.4 buffer ($V_D$). Tested compounds were dissolved first in DMSO (0.5% (V/V) final) and that diluted with PBS pH 7.4 to reach the final concentration 50 μM in the donor well. 300 μl of the donor solution was added to the donor wells ($V_A$) and the donor filter plate was carefully put on the acceptor plate so that coated membrane was "in touch" with both donor solution and acceptor buffer. Test compound diffused from the donor well through the lipid membrane (Area=0.28cm$^2$) to the acceptor well. The concentration of the drug in both donor and the acceptor wells was assessed after 3, 4, 5 and 6 hours of incubation in quadruplicate using the UV plate reader Synergy HT (Biotek, USA) at the maximum absorption wavelength and the standard curve for each compound. The data are presented as the permeability (Pe) according the equation (2)[40,41]:

$$\log P_e = \log \left\{ C \times -ln\left(1 - \frac{[drug]_{acceptor}}{[drug]_{equilibrium}}\right)\right\} \; where \; C = \left(\frac{V_D \times V_A}{(V_D + V_A) \times Area \times time}\right) \quad \text{(Eq. 2)}$$

### 2.6. Monoamine oxidase (MAO) inhibition

The monoamine oxidase exists under the form of two isozymes, the monoamine oxidase A (MAO-A) and the monoamine oxidase B (MAO-B). The inhibition assay for MAO-A and MAO-B was conducted in a white opaque 96-well microplate. The reaction mixture contains 42 nM MAO-A or 200 nM MAO-B and inhibitors ISO1 and ISO2 in final concentrations ranging from 0.1-200 μM in 50 mM potassium phosphate buffer with 20% (v/v) glycerol (pH 7.5). The mixture was pre-incubated at 37°C for 15 min and subsequently substrate kynuramine was added to the final concentration of 50 μM. The whole reaction mixture was incubated at 37°C for 30 min. The deamination product of kynuramine formed during the enzymatic reaction (4-hydroxyquinoline) was determined by fluorescence detection (ex 310/em 400 nm) using microplate reader (Spark multimode microplate reader, Tecan Group Ltd). $IC_{50}$ values were calculated by adjusting the experimental data (enzyme activity versus concentration of inhibitor) to non-linear regression curves using GraphPad Prism 7.
The determination of $K_i$ values was performed for MAO-B enzyme with three different concentrations of each inhibitor: 5 μM, 15 μM and 50 μM. The final concentrations of these inhibitors in the reaction mixture were proposed to cover the entire inhibitory range.

### 2.7. GSK-3β inhibition in vitro assay

Human recombinant GSK-3β, prephosphorylated polypeptide substrate and white 96-well plates were purchased from Millipore (Millipore Iberica S.A.U.). Kinase-Glo Luminescent Kinase Assay was obtained from Promega (Promega Biotech Iberica, SL). Adenosinetriphosphate (ATP) and all other reagents were from Sigma-Aldrich. Assay buffer contained 50 mM 4-(2-hydroxyethyl)-1-piperazineethanesulfonic acid (HEPES) (pH 7.5), 1 mM ethylenediaminetetraacetic acid (EDTA), 1 mM ethylene glycol tetraacetic acid (EGTA), and 15 mM magnesium acetate. The in solution experiments for GSK-3β inhibition analysis were performed by following the method developed by Baki et al[42]. In a typical assay, 10 μL of test compound (dissolved in DMSO at 1 mM concentration and diluted in advance in assay buffer to the desired concentration) and 10 μL (20 ng) of enzyme were added to each well in presence of 20 μL of assay buffer containing GSM substrate and ATP in order to reach a 25 μM and 1 μM final concentration respectively. The final DMSO percentage in the reaction mixture did not exceed 5%. After a 30 min incubation time at 30 °C, the enzymatic reaction was stopped by adding 40 μL of Kinase-Glo reagent. Glow-type luminescence was recorded after 10 min using a VictorX 3 PerkinElmer plate reader. The activity was found proportional to the difference of the total and consumed ATP. The inhibitory activities were calculated on the basis of maximal kinase (average positive) and luciferase activities (average negative) measured in the absence of inhibitor and in the presence of reference compound inhibitor (SB-415286 Merck Millipore, $IC_{50}$ = 55 nM) at total inhibition concentration (5 μM)[43], respectively. The linear regression parameters were determined and the $IC_{50}$ extrapolated (GraphPad Prism 4.0, GraphPad Software Inc.).

### 2.8. Oligomerization Tau protein inhibition

***Tau(306-336) pretreatment***

1 mg of Tau(306-336) (Bachem AG, Germany) was initially dissolved in 1,1,1,3,3,3,-hexafluoro-2-propanol

(HFIP), gently vortexed, sonicated, and kept overnight at room temperature. Subsequently, the sample was aliquoted dried and stored at -20 °C.

***Tau(306-336) peptide aggregation and its inhibition by ThT fluorimetric assay***

Stock solutions of Tau(306-336) peptide (500 μM) were prepared in ultrapure water and immediately used. Stock solution of thioflavin T (ThT, 500 μM) was prepared in 56.3 mM phosphate buffer (PB, pH = 7.4) while stock solution of inhibitors (20 mM) were prepared in DMSO/methanol 10/90. Tau(306-336) aggregation was monitored at 30 °C in black, clear bottom 96-well plate (Greiner) by EnSpire multi-plate reader (Perkin Elmer) using ThT fluorimetric assay[44] with some variations. The excitation and emission wavelengths were set at 446 and 490 nm, respectively. Assay samples were prepared by diluting Tau(306-336) stock solution to 50 μM in the assay mixture which consisted in 20 μM ThT, 48.1 mM PB (final concentrations) in final 100 μL volume (final DMSO and MeOH content: 0.05% and 0.45%, respectively). Inhibition experiments were performed by incubating Tau(306-336) peptide at the given conditions in the presence of tested inhibitors at 50 μM. Fluorescence data were recorded every 10 min overnight with 1 min shaking at 800 rpm prior to each reading. Each inhibitor was assayed in triplicates in at least two independent experiments. Estimation of the inhibitory potency (%) was carried out by comparing fluorescence values at the plateau (average fluorescence intensity value in the 12 - 16 h range). Inhibition % are expressed as the mean ± SEM. Quenching of ThT fluorescence was evaluated by preparing blank solutions containing inhibitor/reference compound and preformed fibrils of Tau(306-336) peptide. Values were averaged and the presented values are expressed as the mean ± SEM.

### 3. *In vivo* assays

#### 3.1. Mice treatment

For behavioural studies, 50 swiss female 10-week-old weighing 25-30 g were used. Memory impairment was induced by the potent antimuscarinic drug scopolamine hydrobromide (0.5 mg/kg, Sigma) dissolved in saline buffer. To explore the potential of BIGI-3h as a cognitive enhancer, the compound was administered at a dose of 1 mg/kg and 10 mg/kg. For comparison, the antiamnesic effect of donepezil was tested (Tocris Bioscience, R&D Systems Inc.) at a dose of 1 mg/kg. Both products, 3h and donepezil, were dissolved in saline buffer containing 3.6% dimethyl sulfoxide. All drug solutions were independently prepared immediately before their use and were administered intraperitoneally in 10 mL/kg of vehicle volume. Different batches of mice were used for each experiment. The animals were distributed into 5 experimental groups (I-V): control groups (I) mice administered 3.6% DMSO in saline buffer, labelled as "control vehicle"; experimental groups (II-V) consisted of (II) mice administered scopolamine dissolved in 3.6% DMSO in saline buffer, labelled as "scop/DMSO," (III) mice co-administered scopolamine plus Donepezil, labelled as "Scopo/donep," (IV) and (V) mice co-administrated scopolamine plus BIGI-3h at 1mg/Kg and 10mg/Kg labelled respectively "Scopo/3h 1" "Scopo/3h 10".

#### 3.2. Object Recognition task (ORT)

Spontaneous object recognition was tested as described previously, 28 with slight modifications. All behavioural experiments took place during the light phase of the 12-h light/ dark cycle between 10 am and 3 pm to minimize the influence of circadian rhythm, and they were conducted with the investigators blind to treatments. Prior to the experiment, mice were handled individually for several days. The ORT was performed in a Plexiglas open field square box (40 Å~ 40 Å~40 cm) with black walls and the floor covered with sawdust. The apparatus was placed in a poorly illuminated (60 lux) and noise-isolated room, and the mice were randomly placed in the experimental room 30 min before beginning the experimental task each day. The behaviour of the animals (time spent exploring the objects and overall locomotor activity) was registered during precise time periods. Briefly, during the 3 consecutive days preceding the test day, mice were habituated to an empty open field box. On the first day animals were allowed to explore the apparatus individually for 5 min in the absence of objects (habituation session). On the second and third days animals were allowed again to explore the apparatus for 10 min in the absence of objects. On the test day, 30 min before the trial session, mice were administered treatments as indicated in the previous section and then subjected to the acquisition trial (T1) and the retention trial (T2). In T1 mice were individually allowed for 6 min to freely explore 2 identical plastic objects placed in the arena, located in symmetric positions at a distance of about 7 cm away from walls. Then the rodents were removed from the environment for an intertrial interval of 1 hr. After the delay period, animals were returned for 5 min (T2) to the bin where 1 of the original objects had been replaced by a novel object (neutral to naive mice) with a different shape, colour and textures than the remaining object previously presented in T1 (familiar object). To score the exploration behaviour of the mice automatically, a camera (Panasonic VDR-D 150E) was mounted above the centre of the arena. The total time spent in active exploration of each object during T1 and T2 was determined with a computerized tracking system and image

analyzer (Ethovision XT, Noldus Information Technology) that tracked the nose, centre and tail points of each mouse[45]. As a criterion of active object exploration, it was measured the time spent by the animal directing its nose to the object at a distance of less than 2 cm and/or touching it with the vibrissae or nose. Mice exploring the objects for less than 15 s within the 6-min period of T1 were discarded from experiments; only mice having a minimal level of total object exploration.

## RESULTS

### 1. Synthesis of the derivate compounds: Examples of compounds BIGI 3a-n

The synthesis of the compounds **BIGI 3a-n** (Figure 1) has been carried out by a straightforward two-step synthesis with good overall yields, as depicted on Figure 1. The reaction of commercial benzylpiperidine **1a-b or 1c**[46] with the benzoyl isocyanate, in $CH_2Cl_2$ at 0°C for 1 hour followed by hydrolysis of obtained compound with NaOH for 48h give the expected compounds **2a-c.** Next, the one-pot Biginelli reaction of compounds **2a-c,** ethyl acetoacetate and different commercial 3-formyl chromone in acetic acid with sodium bisulfate as catalyst, gave the derivate compounds of ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate **BIGI-3a-n.**

The NMR characterizations of the compounds BIGI-3a to BIGI-3n obtained by this process, are described below.

**BIGI-3a** : ethyl 1-((1-benzylpiperidin-4-yl)methyl)-6-methyl-2-oxo-4-(4-oxo-4H-chromen-3-yl)-1,2,3,4-tetrahydropyrimidine-5-carboxylate Yield: 52%.
1H NMR (400 MHz, CDCl3) δ 8.24-8.21 (dd, 1H), 7.65-7.72 (m, 1H), 7.63 (s, 1H), 7.45-7.42 (m, 2H), 7.29-7.26 (m, 8H+ CH2Cl2), 6.02-6.01 (dd, 1H), 5.61-5.60 (dd, 1H), 4.18-4.08 (m, 2H), 4.07-3.99 (m, 1H), 3.47 (s, 2H), 3.36-3.31 (dd, 1H), 2.88-2.77 (dd, 2H), 2.59 (s, 3H), 1.95-1.86 (m, 3H), 1.61-1.47 (m, 3H), 1.33-1.26 (m, 3H), 1.19-1.16 (t, 3H).
13C NMR (400 MHz, CDCl3) δ 176.55, 164.65, 162.42, 155.45, 152.67, 151.65, 150.90, 133.01, 128.28, 127.27, 124.83, 124.44, 122.90, 122.27, 117.15, 98.88, 59.44, 51.88, 45.81, 28.67, 15.54, 13.24.

**BIGI-3b** : ethyl 1-((1-benzylpiperidin-4-yl)methyl)-4-(6-bromo-4-oxo-4H-chromen-3-yl)-6-methyl-2-oxo-1,2,3,4-tetrahydropyrimidine-5-carboxylate Yield: 63%.
1H NMR (400 MHz, CDCl3) δ 8.27-8.26 (d, 1H), 7.69-7.66 (dd, 1H), 7.55 (s, 1H), 7.26-7.16 (m, 5H), 5.97-5.96 (d, 1H), 5.48-5.47 (d, 1H), 4.10-3.99 (m, 2H), 3.52-3.89 (m, 1H), 3.49-3.37 (m, 3H), 2.79-2.75 (m, 2H), 2.51 (s, 3H), 1.79-1.75 (m, 2H), 1.59-1.51 (m, 3H), 1.22-1.08 (m, 7H).
13C NMR (400 MHz, CDCl3) δ 176.17, 165.57, 155.21, 153.48, 153.03, 151.85, 137.83, 136.97, 129.31, 128.47, 128.18, 127.08, 125.25, 123.67, 120.15, 118.88, 99.53, 63.15, 60.43, 53.47, 46.60, 40.58, 36.52, 33.64, 32.07, 31.73, 16.11, 14.30.

**BIGI-3c** : ethyl 1-((1-benzylpiperidin-4-yl)methyl)-4-(7-ethyl-4-oxo-4H-chromen-3-yl)-6-methyl-2-oxo-1,2,3,4-tetrahydropyrimidine-5-carboxylate Yield: 78%.
1H NMR (400 MHz, CDCl3) δ 7.96-7.95 (d, 1H), 7.53 (s, 1H), 7.45-7.43 (dd, 1H), 7.29-7.27 (m, 1H), 7.21-7.15 (m, 6H), 5.97-5.95 (d, 1H), 5.54-5.53 (d, 1H), 4.10-3.92 (m, 3H), 3.37 (s, 2H), 3.28-3.23 (dd, 1H), 2.78-2.66 (m, 4H), 2.51 (s, 3H), 1.84-1.75 (m, 2H), 1.48-1.37 (m, 3H), 1.27-1.17 (m, 5H), 1.11-1.08 (t, 3H).
13C NMR (400 MHz, CDCl3) δ 177.64, 165.73, 154.94, 153.78, 152.55, 151.95, 141.83, 134.30, 129.19, 128.20, 127.11, 123.91, 123.69, 123.06, 118.02, 100.04, 62.91, 60.43, 52.85, 47.08, 46.77, 36.71, 29.72, 28.39, 16.51, 15.54, 14.28

**BIGI-3d** : ethyl 1-(2-(1-benzylpiperidin-4-yl)ethyl)-6-methyl-2-oxo-4-(4-oxo-4H-chromen-3-yl)-1,2,3,4-tetrahydropyrimidine-5-carboxylate Yield: 45%.
1H NMR (400 MHz, CDCl3) δ 8.25-8.22 (d, 1H), 7.72-7.67 (t, 1H), 7.61 (s, 1H), 7.46-7.43 (m, 2H), 7.28-7.26 (m, 4H), 5.97-5.96 (d, 1H), 5.59-5.58 (d, 1H), 4.19-4.06 (m, 2H), 3.98-3.92 (m, 1H), 3.60-3.5 (m, 1H), 3.42 (s, 2H), 2.85-2.81 (d, 2H), 2.6 (s, 3H), 1.85-1.75 (m, 2H), 1.70-1.67 (m, 1H), 1.62-1.53 (m, 2H), 1.42-1.33 (m, 1H), 1.29-1.27 (m, 3H), 1.25-1.15 (m, 3H).
13C NMR (400 MHz, CDCl3) δ 177.52, 165.64, 156.48, 153.58, 152.75, 151.80, 134.00, 129.27, 128.17, 127.06, 125.85, 125.43, 123.95, 123.31, 118.20, 99.70, 63.23, 60.38, 53.56, 53.52, 46.56, 40.46, 36.51, 33.59, 32.11, 31.72, 31.64, 29.70, 16.07, 14.27.

**BIGI-3e** : ethyl 1-(2-(1-benzylpiperidin-4-yl)ethyl)-4-(6-bromo-4-oxo-4H-chromen-3-yl)-6-methyl-2-oxo-1,2,3,4-tetrahydropyrimidine-5-carboxylate Yield: 68%.
1H NMR (400 MHz, CDCl3) δ 8.33 (s, 1H), 7.75-7.73 (d, 1H), 7.61 (s, 1H), 7.33-7.26 (m, 6H), 60.3-6.02 (d, 1H),

5.54-5.53 (d, 1H), 4.14-4.06 (m, 2H), 3.94-3.91 (m, 1H), 3.59-3.50 (m, 1H), 3.42 (s, 2H), 2.84-2.80 (d, 2H), 2.58 (s, 3H), 1.83-1.80 (m, 2H), 1.68-1.57 (m, 4H), 1.24-1.18 (m, 5H), 1.16-1.14 (m, 3H).
13C NMR (400 MHz, CDCl3) δ 176.17, 165.55, 155.19, 153.46, 153.02, 151.80, 137.40, 136.97, 129.39, 128.44, 128.20, 127.18, 125.22, 123.62, 120.14, 118.88, 99.51, 63.04, 60.43, 53.38, 53.34, 46.58, 40.55, 36.45, 33.55, 31.90, 31.56, 16.11, 14.27.

**BIGI-3f** : ethyl 1-(2-(1-benzylpiperidin-4-yl)ethyl)-4-(7-ethyl-4-oxo-4H-chromen-3-yl)-6-methyl-2-oxo-1,2,3,4-tetrahydropyrimidine-5-carboxylate Yield: 57%.
1H NMR (400 MHz, CDCl3) δ 7.96-7.95 (d, 1H), 7.53 (s, 1H), 7.46-7.43 (dd, 1H), 7.29-7.15 (m, 6H), 5.96-5.95 (d, 1H), 5.51-5.50 (d, 1H), 4.10-3.99 (m, 2H), 3.85-3.95 (m, 1H), 3.50-3.45 (m, 1H), 3.34 (s, 2H), 2.77-2.66 (m, 4H), 2.52 (s, 3H), 1.77-1.74 (m, 2H), 1.62-1.59 (m, 1H), 1.54-1.51 (m, 2H), 1.30-1.35 (m, 2H), 1.17-1.23 (m, 6H), 1.11-1.08 (t, 3H).
13C NMR (400 MHz, CDCl3) δ 177.64, 165.68, 154.93, 153.64, 152.66, 151.76, 141.79, 138.00, 134.26, 129.27, 128.17, 127.04, 123.91, 123.74, 123.11, 118.05, 99.83, 63.24, 60.35, 53.57, 53.53, 46.62, 40.45, 36.53, 33.60, 32.15, 31.74, 28.39, 16.07, 15.57, 14.28.

**BIGI-3g** ethyl 1-(2-(1-benzylpiperidin-4-yl)ethyl)-4-(6-chloro-7-methyl-4-oxo-4H-chromen-3-yl)-6-methyl-2-oxo-1,2,3,4-tetrahydropyrimidine-5-carboxylate Yield: 66%.
1H NMR (400 MHz, CDCl3) δ 8.15 (s, 1H), 7.5 (s, 1H), 7.31-7.27 (m, 6H), 6.03-6.02 (d, 1H), 5.54-5.53 (d, 1H), 4.16-4.08 (m, 2H), 3.9 (m, 1H), 3.58-3.48 (m, 3H), 2.90-2.87 (d, 2H), 2.57 (s, 3H), 2.48 (s, 3H), 2.01 (s, 1H), 1.90-1.86 (d, 2H), 1.67-1.58 (m, 3H), 1.27-1.24 (m, 4H), 1.18-1.13 (m, 3H).
13C NMR (400 MHz, CDCl3) δ 176.28, 165.58, 154.71, 153.53, 152.78, 151.70, 143.38, 137.04, 132.17, 129.49, 128.22, 127.26, 125.43, 123.29, 122.98, 119.93, 99.64, 62.82, 60.39, 53.20, 53.15, 46.57, 40.49, 36.42, 33.47, 31.74, 31.38, 20.87, 16.09, 14.26.

**BIGI-3h** : ethyl 1-(2-(1-benzylpiperidin-4-yl)ethyl)-4-(6,8-dichloro-4-oxo-4H-chromen-3-yl)-6-methyl-2-oxo-1,2,3,4-tetrahydropyrimidine-5-carboxylate Yield: 75%.
1H NMR (400 MHz, CDCl3) δ 8.06-8.08 (d, J = 4.0 Hz, 1H), 7.72-7.71 (d, 2H), 7.35-7.31 (m, 4H), 6.12 (d, 1H), 5.51 (d, 1H) 4.18 - 4.06 (m, 2H), 3.88 - 3.86 (m, 1H), 3.65 - 3.57 (m, 1H), 3.16 - 3.13 (m, 2H), 2.56 (s,3H), 2 .21-2.16 (m, 2H), 2.02 (s,3H), 1.76- 1.69 (m, 2H), 1.60 - 1.58 (m, 3H), 1.55-1.57 (m,1H), 1.20 (m, 1H), 1.18 (t, J = 8.0 Hz, 3H).
13C NMR (101 MHz, CDCl3) δ 176.00, 175.75, 165.49, 153.70, 153.20, 151.69, 150.88, 134.24, 131.29, 130.58, 128.77, 125.82, 124.58,124.10, 123.90, 99.51, 61.56, 60.67, 52.32, 46.85, 40.53, 36.04, 32.81, 30.28, 29.95, 22.12,16.26, 14.38.
HRMS ESI-TOF [M+H]+ m/z calcd. for C31H34Cl2N3O5 : 598.1862, found: 598.1870

**BIGI-3i** : ethyl 1-(3-(1-benzylpiperidin-4-yl)propyl)-6-methyl-2-oxo-4-(4-oxo-4H-chromen-3-yl)-1,2,3,4-tetrahydropyrimidine-5-carboxylate Yield: 55%.
1H NMR (400 MHz, CDCl3) δ 8.16-8.14 (dd, 1H), 7.62-7.55 (m, 2H), 7.37-7.33 (m, 2H), 7.24-7.16 (m, 6H), 5.93-5.92 (dd, 1H), 5.52-5.51 (dd, 1H), 4.11-3.99 (m, 2H), 3.75-3.80 (m, 1H), 3.47-3.43 (m, 3H), 2.81-2.78 (m, 2H), 2.53 (s, 3H), 1.89-1.83 (t, 2H), 1.53-1.49 (m, 3H), 1.35-1.45 (m, 1H), 1.18-1.08 (m, 8H).
13C NMR (400 MHz, CDCl3) δ 177.54, 165.69, 156.48, 153.62, 152.75, 151.87, 134.01, 129.39, 127.11, 125.85, 125.43, 123.95, 123.33, 118.18, 99.55, 63.17, 60.39, 53.62, 53.57, 46.59, 42.75, 35.28, 33.42, 31.99, 37.29, 16.17, 14.28.

**BIGI-3j** : ethyl 1-(3-(1-benzylpiperidin-4-yl)propyl)-4-(6-bromo-4-oxo-4H-chromen-3-yl)-6-methyl-2-oxo-1,2,3,4-tetrahydropyrimidine-5-carboxylate Yield: 49%.
1H NMR (400 MHz, CDCl3) δ 8.28-8.27 (d, 1H), 7.68-7.66 (dd, 1H), 7.55 (s, 1H), 7.27-7.18 (m, 7H), 5.86-5.85 (d, 1H), 5.50-5.49 (d, 1H), 4.11-3.99 (m, 2H), 3.78 (m, 1H), 3.45-3.41 (m, 2H), 3.48 (s, 1H), 2.81-2.76 (m, 2H), 2.53 (s, 3H), 1.87-1.81 (t, 2H), 1.53-1.49 (m, 3H), 1.35-1.40 (m, 1H), 1.12-1.09 (m, 8H).
13C NMR (400 MHz, CDCl3) δ 176.25, 165.60, 155.23, 153.46, 152.96, 151.98, 137.01, 129.29, 128.47, 128.16, 127.01, 125.23, 123.62, 120.14, 118.91, 99.32, 63.35, 60.44, 53.76, 46.57, 42.83, 35.46, 33.49, 32.18, 27.28, 16.20, 14.28.

**BIGI-3k** : ethyl 1-(3-(1-benzylpiperidin-4-yl)propyl)-4-(7-ethyl-4-oxo-4H-chromen-3-yl)-6-methyl-2-oxo-1,2,3,4-tetrahydropyrimidine-5-carboxylate Yield: 63%.
1H NMR (400 MHz, CDCl3) δ 7.96-7.95 (d, 1H), 7.53 (s, 1H), 7.45-7.42 (dd, 1H), 7.29-7.19 (m, 8H), 5.91-5.90 (d, 1H), 5.52-5.51 (d, 1H), 4.10-3.99 (m, 2H), 3.77-3.81 (m, 1H), 3.45-3.50 (m, 3H), 2.88-2.81 (d, 2H), 2.72-2.66 (dd, 2H), 2.53 (s, 3H), 1.82-1.90 (m, 2H), 1.52-1.50 (m, 3H), 1.42-1.45 (m, 1H), 1.23-1.08 (m, 12H).

13C NMR (400 MHz, CDCl3) δ 177.68, 165.71, 154.93, 153.65, 152.62, 151.82, 141.79, 134.28, 129.43, 129.37, 128.23, 127.20, 127.19, 123.89, 123.72, 123.09, 118.03, 99.64, 63.17, 63.15, 60.36, 53.63, 53.59, 46.62, 42.71, 35.31, 33.36, 31.94, 31.93, 31.91, 31.88, 29.72, 28.39, 27.28, 16.16, 15.54, 14.28.

**BIGI-3l** ethyl 1-(3-(1-benzylpiperidin-4-yl)propyl)-4-(6-chloro-7-methyl-4-oxo-4H-chromen-3-yl)-6-methyl-2-oxo-1,2,3,4-tetrahydropyrimidine-5-carboxylate Yield: 62%.
1H NMR (400 MHz, CDCl3) δ 8.09 (s, 1H), 7.51 (s, 1H), 7.24-7.16 (m, 7H), 5.88-5.87 (d, 1H), 5.50-5.49 (d, 1H), 4.10-3.99 (m, 2H), 3.78-3.58 (m, 1H), 3.47-3.42 (m, 3H), 2.79-2.77 (d, 2H), 2.52 (s, 3H), 2.41 (s, 3H), 1.87-1.82 (t, 2H), 1.54-1.49 (m, 3H), 1.35-1.45 (m, 1H), 1.18-1.08 (m, 9H).
13C NMR (400 MHz, CDCl3) δ 176.36, 165.64, 154.75, 153.52, 152.76, 151.89, 143.41, 132.20, 129.29, 128.17, 127.02, 125.45, 123.31, 122.99, 119.93, 99.44, 63.33, 60.40, 53.71, 46.54, 42.80, 35.46, 33.46, 32.15, 27.28, 20.87, 16.18, 14.28.

**BIGI-3m** : ethyl 1-(3-(1-benzylpiperidin-4-yl)propyl)-4-(6,8-dichloro-4-oxo-4H-chromen-3-yl)-6-methyl-2-oxo-1,2,3,4-tetrahydropyrimidine-5-carboxylate Yield: 49%.
1H NMR (400 MHz, CDCl3) δ 8.03-8.02 (d, 1H), 7.66-7.64 (dd , 2H), 7.25-7.18 (m, 8H), 5.825-5.817 (d, 1H), 5.488-5.480 (d, 1H), 4.12-4.00 (m, 2H), 3.70-3.80 (m, 1H), 3.47-3.45 (m, 3H), 2.87-2.81 (m, 2H), 2.53 (s, 3H), 1.80-1.95 (m, 2H), 1.50-1.65 (m, 3H), 1.45-1.48 (m, 1H), 1.18-1.35 (m, 10H).
13C NMR (400 MHz, CDCl3) δ 175.82, 165.46, 153.37, 152.99, 152.12, 150.80, 134.17, 131.21, 129.43, 128.24, 127.19, 125.69, 124.48, 124.00, 123.81, 99.05, 63.20, 60.52, 53.59, 46.56, 42.94, 35.35, 33.42, 31.94, 29.71, 27.28, 16.22, 14.30.

**BIGI-3n** : ethyl 1-(3-(1-benzylpiperidin-4-yl)propyl)-4-(6-isopropyl-4-oxo-4H-chromen-3-yl)-6-methyl-2-oxo-1,2,3,4-tetrahydropyrimidine-5-carboxylate Yield: 45%.
1H NMR (400 MHz, CDCl3) δ 7.98-7.97 (d, 1H), 7.54 (d, 1H), 4.46-7.50 (dd, 1H), 7.32-7.20 (m, 7H), 6.18-6.17 (d, 1H), 5.49-5.48 (d, 1H), 4.10-3.99 (m, 2H), 3.9 (s, 2H), 3.70-3.80 (m, 1H), 3.45-3.50 (m, 1H), 3.23-3.20 (m, 2H), 2.98-2.94 (m, 1H), 2.5 (s, 3H), 2.30-2.35 (m, 2H), 1.97 (s, 5H), 1.60-1.65 (m, 2H), 1.48-1.08 (m, 20H).
13C NMR (400 MHz, CDCl3) δ 177.63, 165.65, 154.93, 153.87, 152.73, 151.41, 146.44, 133.04, 131.10, 130.91, 129.03, 129.82, 123.70, 123.05, 122.46, 118.06, 99.72, 80.62, 80.40, 51.80, 46.82, 42.58, 33.86, 32.47, 30.32, 29.70, 29.51, 29.40, 26.93, 23.94, 23.91, 16.16, 14.26.

## 2. Biological activities

### 2.1. Calcium channel antagonist activity, cholinesterase inhibition activity and antioxidant activity

The blocking effect of the depolarization-induced $Ca^{2+}$ uptake was investigated in SH-SY5Y neuroblastoma cells stimulated with an extracellular concentration of $K^+$ 70 mM, which induces the cell $Ca^{2+}$ levels elevation by opening voltage-gated $Ca^{2+}$ channels (VGCC), monitored with the $Ca^{2+}$-sensitive fluorescent dye Fluo-4. Nifedipine was used as positive standard. $IC_{50}$ results of the compounds BIGI-3a-n are shown in Table 1 below. Interestingly, most compounds displayed a $Ca^{2+}$ channel blockade better than Nifedipine.
Then, the cholinesterase (hChE) inhibition capacity of compounds BIGI-3a-n following Ellman's protocol[31] has been evaluated (see Table 1). Interestingly several compounds showed an inhibition against hAChE and hBuChE in micromolar range and particularly compounds BIGI-3e and BIGI-3h which showed a submicromolar activity against AChE with $IC_{50}$= 0.06 μM. The best active compound against BuChE was BIGI-3k with $IC_{50}$ equal to 2.95 μM.
The antioxidant activity was then evaluated by ORAC[47] assay and showing that all compounds exhibit a strong antioxidant power ranging from 1.68 to 3.65 TE compared to those of melatonin and Ferulic acid which showing respectively 2.45 and 3.7TE (Table 1).

**Table 1.** Calcium channel blockade, ChE inhibition, and ORAC analysis of compounds BIGI-3a-n and standard compounds.

| Compounds | $IC_{50}$ (μM) Calcium channels blockade | AChE ($IC_{50}$ μM) | BChE ($IC_{50}$ μM) | ORAC |
|---|---|---|---|---|
| BIGI-3a | 14 ± 3 | n.d. | 14.8 ± 0.7 | 3.29 ± 0.07 |
| BIGI-3b | 6 ± 3 | n.d. | n.d. | 2.06 ± 0.17 |
| BIGI-3c | 3.0 ± 0.9 | 6.08 ± 0.38 | 36.1 ± 3.1 | 3.51 ± 0.09 |

(continued)

| Compounds | $IC_{50}$ (μM) Calcium channels blockade | AChE ($IC_{50}$ μM) | BChE ($IC_{50}$ μM) | ORAC |
|---|---|---|---|---|
| BIGI-3d | 9 ± 2 | 0.23 ± 0.01 | 6.58 ± 0.34 | 2.53 ± 0.17 |
| BIGI-3e | 1.9 ± 0.6 | 0.06 ± 0.00 | 8.4 ± 0.34 | 3.26 ± 0.12 |
| BIGI-3f | 2.2 ± 0.6 | 0.30 ± 0.01 | 5.96 ± 0.22 | 2.20 ± 0.05 |
| BIGI-3g | 1.7 ± 0.6 | 0.29 ± 0.01 | 15.7 ± 0.9 | 2.56 ± 0.13 |
| BIGI-3h | 1.4 ± 0.4 | 0.06 ± 0.00 | 17.3 ± 0.9 | 3.65 ± 0.07 |
| BIGI-3i | 8 ± 1 | 0.15 ± 0.00 | 4.29 ± 0.09 | 3.09 ± 0.07 |
| BIGI-3j | 3.8 ± 0.1 | 0.18 ± 0.01 | 6.51 ± 0.45 | 3.24 ± 0.02 |
| BIGI-3k | 1.4 ± 0.5 | 0.50 ± 0.02 | 2.95 ± 0.14 | 2.75 ± 0.16 |
| BIGI-3l | 3.2 ± 0.2 | 0.17 ± 0.01 | 6.26 ± 0.31 | 1.68 ± 0.18 |
| BIGI-3m | 5 ± 2 | 0.34 ± 0.02 | 14.4 ± 1.2 | 3.29± 0.10 |
| BIGI-3n | 1.7 ± 0.4 | 0.58 ± 0.02 | 4.35 ± 0.23 | 2.45± 0.04 |
| Nifedipine | 6.2 ± 0.2 | -[a] | -[a] | -[a] |
| Melatonin | -[a] | -[a] | -[a] | 2.5 ± 0.1 |
| Ferulic acid | -[a] | -[a] | -[a] | 3.7 ± 0.2 |

Each $IC_{50}$ value is the mean ± SEM of quadruplicate of at least three different experiments; *not evaluated.*

The most balanced compounds taking into account of the calcium channel blockade and ChE inhibition were compounds BIGI-3e, BIGI-3h and BIGI-3k. In fact, BIGI-3h and BIGI-3k were the best calcium channel blockers and the best active compounds against AchE and BuChE respectively when BIGI-3k was the best active compound with BIGI-3h showing a strong calcium channel blockade activity.

These 3 selected BIGI compounds were submitted to additional biological evaluation: kinetic study, Inhibition of $A\beta_{1-42}$ self-aggregation and their capacity to cross the blood brain barrier.

**2.2. Inhibition of AChE hydrolysis: Kinetic studies of compounds BIGI-3h and BIGI-3e**

To obtain insight into the interactions of BIGI-3h and BIGI-3e with human AChE, a kinetic study was conducted. Substrate concentration - enzyme velocity curves were recorded in the presence of different concentrations of the tested compounds. Graphical analysis of Lineweaver-Burk reciprocal plots (Figures 2 and 3) showed identical mode of interaction with AChE for both inhibitors, the intersection of lines is located just above the x-axis. Km was very slightly increased, whereas value of Vmax decreased with higher concentration of compounds. The type of inhibition and corresponding inhibition constants (Ki and Ki') were determined by the nonlinear regression analysis of data. Results for each type model of inhibition (competitive, non-competitive, uncompetitive and mixed) were compared with sum-of-squares F-test. Analysis indicated mixed non-competitive type of inhibition ($p<0.05$), consistent with graphical representation in Figures 2 and 3. BIGI-3h and BIGI-3e bind to both free AChE and AChE-substrate complex, affecting the binding of substrate in the active site of enzyme, interacting with its allosteric peripheral anionic site. Interaction with this site causes conformational changes of the enzyme resulting also in changes of its active site. A Ki value of 21.46 ± 5.19 nM and Ki' of 77.57 ± 16.42 nM were measured for BIGI-3h and a Ki value of 51.01 ± 12.74 nM and Ki' of 89.53 ± 12.47 nM for BIGI-3e, respectively. Measured values are consistent with the $IC_{50}$ values for AChE. It appears that both tested compounds tend to bind free AChE more than enzyme-substrate complex.

**2.3. Inhibition of $A\beta_{1-42}$ self-aggregation and Prediction of blood-brain barrier penetration**

The inhibitory activity of compounds BIGI-3e, BIGI-3h and BIGI-3k against the spontaneous aggregation of $A\beta_{1-42}$ was determined *in vitro* using a thioflavin T (ThT)-based fluorometric assay[35]. The 3 compounds showed interestingly $A\beta_{1-42}$ antiaggregating activity (Table 2), displaying percentages of inhibition in the range 63-66% when tested at equimolar concentration with $A\beta_{1-42}$ ([Inhibitor]=[$A\beta_{1-42}$]=50 μM). These data further confirm that these compounds are able to directly inhibit the formation of $A\beta$ toxic species, as already observed in cell based-assay.

Concerning the prediction of blood-brain barrier (BBB) penetration using the PAMPA assay [38, 39], compound BIGI-3h showed the best ability to cross the BBB (Table 2).

**Table 2.** Percentage of inhibition of A$\beta_{1-42}$ aggregation and ability to cross blood-brain barrier for compounds BIGI-3e, BIGI-3k and BIGI-3h

| Compounds | % inhibition A$\beta_{1-42}$ aggregation | CNS |
|---|---|---|
| BIGI-3e | 63.0 ± 2.1 | CNS (+/-) |
| BIGI-3k | 63.8 ± 0.8 | CNS (-) |
| BIGI-3h | 66.0 ± 0.7 | CNS (+) |

Each % value is the mean ± SEM of quadruplicate of at least three different experiments

As BIGI-3h contains a stereocenter, the activity of the two enantiomers was studied regarding AChE inhibition, calcium channel blockade, A$\beta_{1-42}$ aggregation and their ability to cross Blood Brain barrier. The two enantiomers ISO1 (+) and ISO 2(-), separated using chiral chromatographic column, showed very few differences of activity compared to the racemate of BIGI-3h as shown in Table 3.

**Table 3.** AChE inhibition, Calcium channel blockade, % inhibition A$\beta_{1-42}$ aggregation and ability to cross BBB for compound BIGI-3h and its isomers

| Compounds | AChE ($IC_{50}$ μM) | $IC_{50}$ (μM) Calcium channels blockade | % Inhibition A$\beta_{1-42}$ | Pe ± SEM ($*10^{-6}$ cm $s^{-1}$) |
|---|---|---|---|---|
| BIGI-3h | 0.06 ± 0.00 | 1.4 ± 0.4 | 63.6 ± 1.3 | 4.0 ± 0.3 |
| BIGI-3h ISO1 (+) | 0.09 ± 0.00 | 5 ± 2 | 66.5 ± 0.9 | 6.7 ± 0.4 |
| BIGI-3h ISO2 (-) | 0.07 ± 0.00 | 1.6 ± 0.4 | 63.5 ± 0.2 | 7.5± 0.8 |

Each $IC_{50}$ and % values are the mean ± SEM of quadruplicate of at least three different experiments.

### 2.4. Monoamine oxidase (MAO) inhibition, GSK-3β inhibition, Tau (T) self-aggregation inhibition

The MAO inhibition, the GSK-3β inhibition and the Tau (306-336) self-aggregation inhibition were evaluated for compound BIGI-3h and its isomers (+) and (-) (Table 4).

**Table 4.** MAO inhibition, GSK-3β inhibition and percentage of inhibition of $T_{(306-336)}$ peptide self - aggregation of BIGI-3h and its isomers

| Compounds | MAO A ($IC_{50}$ μM) | MAO B ($IC_{50}$ μM) | GSK-3β inhibition ($IC_{50}$ μM) | Inhibition of $T_{(306-336)}$ peptide self - aggregation (%) |
|---|---|---|---|---|
| BIGI-3h | - | - | 29.1 ± 0.5 | 73.7 ± 5.4 |
| BIGI-3h ISO1 (+) | 75.63 ± 35.77 | 11.20 ± 1.095 | 10.6 ± 1.5 | 78.3 ± 3.0 |
| BIGI-3h ISO2 (-) | 195.20 ± 505.3 | 12.34 ± 1.393 | 24.3 ± 1.2 | 75.3 ± 1.6 |
| Clorgylin | 0.045 ± 0.003 | - | | |
| Pargylin | | 0.227 ± 0.029 | | |
| SB-415286 | | | 0.055 | |

Each $IC_{50}$ and % values are the mean ± SEM of quadruplicate of at least three different experiments.

#### 2.4.1. MAO inhibition

MAO isozymes are involved in neurodegenerative diseases. Hyperactivity of MAO enzymes, decreasing the concentration of dopaminergic and serotoninergic neurotransmitters, has been observed in AD patients[48,49]. MAO-A

contributes to toxin-induced apoptosis and striatal damage of neuronal cells. The expression of MAO-B in neuronal tissues increases with aging, resulting in increased dopamine metabolism and higher levels of hydrogen peroxide and oxidative free radicals. MAO-B is therefore thought to play a pivotal role in the progression of AD[48,50].

The MAO-A and MAO-B inhibitory activity of BIGI-3h ISO1 and BIGI-3h ISO2 was investigated and showed a modest inhibition of MAO A, whereas an interesting and selective activity against MAO B was observed, with comparable $IC_{50}$ equal to 11.2 and 12.34 $\mu$M respectively.

**2.4.2. Inhibitory activity toward GSK-3β**

Compounds BIGI-3h, BIGI-3h ISO1 and BIGI-3h ISO2 were evaluated for their ability to inhibit GSK-3β, using a luminescence assay[42], in comparison with SB-415286[51], a well-known GSK3β inhibitor. As reported in Table 4, BIGI-3h and its isomer BIGI-3h ISO2 showed a comparable $IC_{50}$ equal to 29.1 and 24.3 $\mu$M respectively, when BIGI-3h ISO1 displayed a more interesting $IC_{50}$ equal to 10.6$\mu$M, in comparison with SB-415286 $IC_{50}$ equal to 55 nM. These inhibitions potencies in micromolar range are in line with the activity of other GSK-3β inhibitors based on multipotent inhibitors[52]. In addition evidences from in vivo studies indicate that micromolar concentration could be more adequate to produce therapeutic effects in the brain without affecting GSK-3β activity in peripheral tissues[53].

**2.4.3. Inhibition of Tau(306-336) peptide aggregation**

The inhibition of Tau(306-336) peptide aggregation was then evaluated. Inhibition experiments were performed by incubating Tau(306-336) peptide 50 $\mu$M in the presence of potential tested inhibitors at 50 $\mu$M (1:1 ratio). Fluorescence data were recorded every 10 min overnight with 1 min shaking at 800 rpm prior to each reading.

BIGI-3h showed an interesting inhibitory pattern being able to delay lag phase ($t_{1/2}$ from 4.5h to 6.3h) (Figure 4) and inhibit tau(306-336) self-aggregation by 73.7% (Table 4), while the anti-cholinesterase drug tacrine was not able to significantly interfere with Tau(306-336) aggregation. Single enantiomers were assayed in the same experimental conditions. No significant difference in the anti-tau properties was encountered for single enantiomers compared to the racemic mixture.

***3. In vivo* assays**

An *in vivo* assay with compound BIGI-3h was carried out to get the proof-of-concept of this multitarget directed ligand for Alzheimer disease. The novel object recognition task was used. This test widely used and constitutes a valuable measure of cognition[54].

Treatment of mice (0.5 mg/Kg) with Scopolamine, a compound that impairs cognitive abilities, induced significant decrease of the discrimination index compared to control group. In fact Scopolamine produces a memory acquisition deficit constituting, thus, an appropriate model for evaluating short-term memory. The results at Figure 5 showed that a treatment with either donepezil (done/scop group), a commercially available drug for the treatment of Alzheimer's disease, or BIGI-3h 1mg/Kg (scopo/bigi1 group) reversed the short-term memory impairment induced by scopolamine. Moreover, mice treated with 10 mg/kg of compound BIGI-3h after scopolamine showed a significantly improved discrimination index. The recognition memory deficits caused by scopolamine (0.5 mg g/Kg) injection are thus restored at an enhanced level by compound BIGI-3h at 10 mg/kg. In conclusion, Figure 5 shows that BIGI-3h at 10 mg/kg is able to reverse scopolamine-induced cognitive impairment and is more effective to reverse the effects of scopolamine compared to Donepezil.

**BIBLIOGRAPHIE**

(1) Querfurth, H. W.; LaFerla, F. M. Alzheimer's Disease. N. Engl. J. Med. 2010, 362 (4), 329-344.

(2) Bartus, R. T.; Dean, R. L.; Beer, B.; Lippa, A. S. The Cholinergic Hypothesis of Geriatric Memory Dysfunction. Science 1982, 217 (4558), 408-414.

(3) Liu, Z.; Zhang, A.; Sun, H.; Han, Y.; Kong, L.; Wang, X. Two Decades of New Drug Discovery and Development for Alzheimer's Disease. RSC Adv. 2017, 7 (10), 6046-6058.

(4) Semagacestat's fall: where next for AD therapies? (accessed Oct 9, 2018).

(5) Kumar, A.; Singh, A.; Ekavali, null. A Review on Alzheimer's Disease Pathophysiology and Its Management: An

Update. Pharmacol. Rep. PR 2015, 67 (2), 195-203.

(6) Godyń, J.; Jończyk, J.; Panek, D.; Malawska, B. Therapeutic Strategies for Alzheimer's Disease in Clinical Trials. Pharmacol. Rep. 2016, 68 (1), 127-138.

(7) Li, C.; Götz, J. Tau-Based Therapies in Neurodegeneration: Opportunities and Challenges. Nat. Rev. Drug Discov. 2017, 16 (12), 863-883.

(8) Avila, J.; Perez, M.; Lim, F.; Gómez-Ramos, A.; Hernández, F.; Lucas, J. J. Tau in Neurodegenerative Diseases: Tau Phosphorylation and Assembly. Neurotox. Res. 2004, 6 (6), 477-482.

(9) Villarroya, M.; Garcia, A. G.; Marco-Contelles, J.; López, M. G. An Update on the Pharmacology of Galantamine. Expert Opin. Investig. Drugs 2007, 16 (12), 1987-1998.

(10) Godyń, J.; Jończyk, J.; Panek, D.; Malawska, B. Therapeutic Strategies for Alzheimer's Disease in Clinical Trials. Pharmacol. Rep. 2016, 68 (1), 127-138.

(11) Chen, C.-H.; Zhou, W.; Liu, S.; Deng, Y.; Cai, F.; Tone, M.; Tone, Y.; Tong, Y.; Song, W. Increased NF-KB Signalling up-Regulates BACE1 Expression and Its Therapeutic Potential in Alzheimer's Disease. Int. J. Neuropsychopharmacol. 2012, 15 (1), 77-90.

(12) Cho, H. J.; Jin, S. M.; Youn, H. D.; Huh, K.; Mook-Jung, I. Disrupted Intracellular Calcium Regulates BACE1 Gene Expression via Nuclear Factor of Activated T Cells 1 (NFAT 1) Signaling. Aging Cell 7 (2), 137-147.

(13) Hayley, M.; Perspicace, S.; Schulthess, T.; Seelig, J. Calcium Enhances the Proteolytic Activity of BACE1: An in Vitro Biophysical and Biochemical Characterization of the BACE1-Calcium Interaction. Biochim. Biophys. Acta 2009, 1788 (9), 1933-1938.

(14) Oulès, B.; Del Prete, D.; Greco, B.; Zhang, X.; Lauritzen, I.; Sevalle, J.; Moreno, S.; Paterlini-Bréchot, P.; Trebak, M.; Checler, F.; et al. Ryanodine Receptor Blockade Reduces Amyloid-β Load and Memory Impairments in Tg2576 Mouse Model of Alzheimer Disease. J. Neurosci. Off. J. Soc. Neurosci. 2012, 32 (34), 11820-11834.

(15) Small, D. H.; Gasperini, R.; Vincent, A. J.; Hung, A. C.; Foa, L. The Role of Aβ-Induced Calcium Dysregulation in the Pathogenesis of Alzheimer's Disease. J. Alzheimers Dis. 2009, 16 (2), 225-233.

(16) Avila, J.; Perez, M.; Lim, F.; Gómez-Ramos, A.; Hernández, F.; Lucas, J. J. Tau in Neurodegenerative Diseases: Tau Phosphorylation and Assembly. Neurotox. Res. 2004, 6 (6), 477-482.

(17) Chakroborty, S.; Stutzmann, G. E. Calcium Channelopathies and Alzheimer's Disease: Insight into Therapeutic Success and Failures. Eur. J. Pharmacol. 2014, 739, 83-95.

(18) von Bernhardi, R.; Eugenín, J. Alzheimer's Disease: Redox Dysregulation As a Common Denominator for Diverse Pathogenic Mechanisms. Antioxid. Redox Signal. 2011, 16 (9), 974-1031.

(19) Praticò, D. Oxidative Stress Hypothesis in Alzheimer's Disease: A Reappraisal. Trends Pharmacol. Sci. 2008, 29 (12), 609-615.

(20) Praticò, D.; Sung, S. Lipid Peroxidation and Oxidative Imbalance: Early Functional Events in Alzheimer's Disease. J. Alzheimers Dis. 2004, 6 (2), 171-175.

(21) Federico, A.; Cardaioli, E.; Da Pozzo, P.; Formichi, P.; Gallus, G. N.; Radi, E. Mitochondria, Oxidative Stress and Neurodegeneration. J. Neurol. Sci. 2012, 322 (1-2), 254-262.

(22) Yan, M. H.; Wang, X.; Zhu, X. Mitochondrial Defects and Oxidative Stress in Alzheimer Disease and Parkinson Disease. Free Radic. Biol. Med. 2013, 62, 90-101.

(23) Greenough, M. A.; Camakaris, J.; Bush, A. I. Metal Dyshomeostasis and Oxidative Stress in Alzheimer's Disease. Neurochem. Int. 2013, 62 (5), 540-555.

(24) Candore, G.; Bulati, M.; Caruso, C.; Castiglia, L.; Colonna-Romano, G.; Di Bona, D.; Duro, G.; Lio, D.; Matranga, D.; Pellicano, M.; et al. Inflammation, Cytokines, Immune Response, Apolipoprotein E, Cholesterol, and Oxidative Stress in Alzheimer Disease: Therapeutic Implications. Rejuvenation Res. 2010, 13 (2-3), 301-313.

(25) Lee, Y.-J.; Han, S. B.; Nam, S.-Y.; Oh, K.-W.; Hong, J. T. Inflammation and Alzheimer's Disease. Arch. Pharm. Res. 2010, 33 (10), 1539-1556.

(26) Alper, G.; Girgin, F. K.; Ozgönül, M.; Menteş, G.; Ersöz, B. MAO Inhibitors and Oxidant Stress in Aging Brain Tissue. *Eur. Neuropsychopharmacol. J. Eur. Coll. Neuropsychopharmacol.* **1999,** *9* (3), 247-252.

(27) Marin, D. B.; Bierer, L. M.; Lawlor, B. A.; Ryan, T. M.; Jacobson, R.; Schmeidler, J.; Mohs, R. C.; Davis, K. L. L-Deprenyl and Physostigmine for the Treatment of Alzheimer's Disease. Psychiatry Res. 1995, 58(3), 181-189.

(28) Rosini, M.; Simoni, E.; Milelli, A.; Minarini, A.; Melchiorre, C. Oxidative Stress in Alzheimer's Disease: Are We Connecting the Dots? J. Med. Chem. 2014, 57 (7), 2821-2831.

(29) Carling, W. R.; Moore, K. W. Imidazolone and Oxazolone Derivatives as Dopamine Antagonists. US5698573A, December 16, 1997.

(30) Gonzalez-Lafuente, L.; Egea, J.; León, R.; Martinez-Sanz, F. J.; Monjas, L.; Perez, C.; Merino, C.; Garcia-De Diego, A. M.; Rodriguez-Franco, M. I.; Garcia, A. G.; et al. Benzothiazepine CGP37157 and Its Isosteric 2'-Methyl Analogue Provide Neuroprotection and Block Cell Calcium Entry. ACS Chem. Neurosci. 2012, 3 (7), 519-529.

(31) Ellman, G. L.; Courtney, K. D.; Andres jr., V.; Featherstone, R. M. A New and Rapid Colorimetric Determination of Acetylcholinesterase Activity. Biochem. Pharmacol. 1961, 7 (2), 88-95.

(32) Nepovimova, E.; Korabecny, J.; Dolezal, R.; Babkova, K.; Ondrejicek, A.; Jun, D.; Sepsova, V.; Horova, A.; Hrabinova, M.; Soukup, O.; et al. Tacrine-Trolox Hybrids: A Novel Class of Centrally Active, Nonhepatotoxic Multi-Target-Directed Ligands Exerting Anticholinesterase and Antioxidant Activities with Low In Vivo Toxicity. J. Med. Chem. 2015, 58 (22), 8985-9003.

(33) Ou, B.; Hampsch-Woodill, M.; Prior, R. L. Development and Validation of an Improved Oxygen Radical Absorbance Capacity Assay Using Fluorescein as the Fluorescent Probe. J. Agric. Food Chem. 2001, 49 (10), 4619-4626.

(34) Dávalos, A.; Gómez-Cordovés, C.; Bartolomé, B. Extending Applicability of the Oxygen Radical Absorbance Capacity (ORAC-Fluorescein) Assay. J. Agric. Food Chem. 2004, 52 (1), 48-54.

(35) Bartolini, M.; Bertucci, C.; Bolognesi, M. L.; Cavalli, A.; Melchiorre, C.; Andrisano, V. Insight into the Kinetic of Amyloid Beta (1-42) Peptide Self-Aggregation: Elucidation of Inhibitors' Mechanism of Action. Chembiochem Eur. J. Chem. Biol. 2007, 8 (17), 2152-2161.

(36) Naiki, H.; Higuchi, K.; Hosokawa, M.; Takeda, T. Fluorometric Determination of Amyloid Fibrils in Vitro Using the Fluorescent Dye, Thioflavin T1. Anal. Biochem. 1989, 177 (2), 244-249.

(37) LeVine, H. 3rd. Thioflavine T Interaction with Synthetic Alzheimer's Disease Beta-Amyloid Peptides: Detection of Amyloid Aggregation in Solution. Protein Sci. Publ. Protein Soc. 1993, 2 (3), 404-410.

(38) Lemes, L. F. N.; de Andrade Ramos, G.; de Oliveira, A. S.; da Silva, F. M. R.; de Castro Couto, G.; da Silva Boni, M.; Guimarães, M. J. R.; Souza, I. N. O.; Bartolini, M.; Andrisano, V.; et al. Cardanol-Derived AChE Inhibitors: Towards the Development of Dual Binding Derivatives for Alzheimer's Disease. Eur. J. Med. Chem. 2016, 108, 687-700.

(39) Di, L.; Kerns, E. H.; Fan, K.; McConnell, O. J.; Carter, G. T. High Throughput Artificial Membrane Permeability Assay for Blood-Brain Barrier. Eur. J. Med. Chem. 2003, 38 (3), 223-232.

(40) Sugano, K.; Hamada, H.; Machida, M.; Ushio, H. High Throughput Prediction of Oral Absorption: Improvement of the Composition of the Lipid Solution Used in Parallel Artificial Membrane Permeation Assay. J. Biomol. Screen.

2001, 6 (3), 189-196.

(41) Wohnsland, F.; Faller, B. High-Throughput Permeability PH Profile and High-Throughput Alkane/Water Log P with Artificial Membranes. J. Med. Chem. 2001, 44 (6), 923-930.

(42) Baki, A.; Bielik, A.; Molnár, L.; Szendrei, G.; Keserü, G. M. A High Throughput Luminescent Assay for Glycogen Synthase Kinase-3beta Inhibitors. Assay Drug Dev. Technol. 2007, 5 (1), 75-83.

(43) Coghlan, M. P.; Culbert, A. A.; Cross, D. A.; Corcoran, S. L.; Yates, J. W.; Pearce, N. J.; Rausch, O. L.; Murphy, G. J.; Carter, P. S.; Roxbee Cox, L.; et al. Selective Small Molecule Inhibitors of Glycogen Synthase Kinase-3 Modulate Glycogen Metabolism and Gene Transcription. Chem. Biol. 2000, 7 (10), 793-803.

(44) Gandini, A.; Bartolini, M.; Tedesco, D.; Martinez-Gonzalez, L.; Roca, C.; Campillo, N. E.; Zaldivar-Diez, J.; Perez, C.; Zuccheri, G.; Miti, A.; et al. Tau-Centric Multitarget Approach for Alzheimer's Disease: Development of First-in-Class Dual Glycogen Synthase Kinase 3β and Tau-Aggregation Inhibitors. J. Med. Chem. 2018, 61 (17), 7640-7656.

(45) Small, D. H.; Gasperini, R.; Vincent, A. J.; Hung, A. C.; Foa, L. The Role of Aβ-Induced Calcium Dysregulation in the Pathogenesis of Alzheimer's Disease. J. Alzheimers Dis. 2009, 16 (2), 225-233.

(46) Choi, J. Y.; Calvet, C. M.; Gunatilleke, S. S.; Ruiz, C.; Cameron, M. D.; McKerrow, J. H.; Podust, L. M.; Roush, W. R. Rational Development of 4-Aminopyridyl-Based Inhibitors Targeting Trypanosoma Cruzi CYP51 as Anti-Chagas Agents. J. Med. Chem. 2013, 56 (19), 7651-7668.

(47) Dávalos, A.; Gómez-Cordovés, C.; Bartolomé, B. Extending Applicability of the Oxygen Radical Absorbance Capacity (ORAC-Fluorescein) Assay. J. Agric. Food Chem. 2004, 52 (1), 48-54.

(48) Kumar, B.; Gupta, V. P.; Kumar, V. A Perspective on Monoamine Oxidase Enzyme as Drug Target: Challenges and Opportunities. Curr. Drug Targets 2017, 18 (1), 87-97.

(49) Knez, D.; Sova, M.; Košak, U.; Gobec, S. Dual Inhibitors of Cholinesterases and Monoamine Oxidases for Alzheimer's disease. Future Med. Chem. 2017, 9 (8), 811-832.

(50) Reis, J.; Cagide, F.; Valencia, M. E.; Teixeira, J.; Bagetta, D.; Perez, C.; Uriarte, E.; Oliveira, P. J.; Ortuso, F.; Alcaro, S.; et al. Multi-Target-Directed Ligands for Alzheimer's Disease: Discovery of Chromone-Based Monoamine Oxidase/Cholinesterase Inhibitors. Eur. J. Med. Chem. 2018, 158, 781-800.

(51) Cohen, P.; Goedert, M. GSK3 Inhibitors: Development and Therapeutic Potential. Nat. Rev. Drug Discov. 2004, 3 (6), 479-487.

(52) Prati, F.; De Simone, A.; Bisignano, P.; Armirotti, A.; Summa, M.; Pizzirani, D.; Scarpelli, R.; Perez, D. I.; Andrisano, V.; Perez-Castillo, A.; et al. Multitarget Drug Discovery for Alzheimer's Disease: Triazinones as BACE-1 and GSK-3β Inhibitors. Angew. Chem. Int. Ed. 2015, 54 (5), 1578-1582.

(53) Avrahami, L.; Licht-Murava, A.; Eisenstein, M.; Eldar-Finkelman, H. GSK-3 Inhibition: Achieving Moderate Efficacy with High Selectivity. Biochim. Biophys. Acta BBA - Proteins Proteomics 2013, 1834 (7), 1410-1414.

(54) Antunes, M.; Biala, G. The Novel Object Recognition Memory: Neurobiology, Test Procedure, and Its Modifications. Cogn. Process. 2012, 13 (2), 93-110.

## Claims

1. A compound of formula (I):

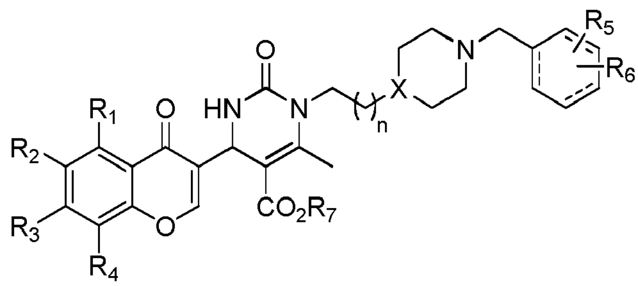

(I)

including any stereochemically isomeric form thereof,
wherein:

$R_1$, $R_2$, $R_3$ and $R_4$ independently of each other, are H, halogen, substituted or unsubstituted $C_1$-$C_6$ alkyl, $NO_2$, OH, O-methyl, CN, acyl, alkoxycarbonyl, SH or NR'R" with R' and R" being either or independently of each other hydrogen, alkyl, substituted or not aryl, or R' and R" forming together a cycloalkyl;
$R_5$ and $R_6$, independently of each other, are H, halogen, substituted or unsubstituted $C_1$-$C_6$ alkyl, cycloalkyl, substituted or not aryl, $NO_2$, OH, CN, acyl, alkoxy, alkoxycarbonyl, SH;
$R_7$ is a substituted or unsubstituted $C_1$-$C_6$ alkyl or a substituted or unsubstituted aryl;
X is N or CH;
n is 0, 1 or 2;
the symbol - - - indicates a single or double bond;
or a pharmaceutically acceptable salt thereof.

**2.** Compound according to claim 1 wherein $R_5$ and $R_6$ are H.

**3.** Compound according to claim 1 or 2 wherein $R_7$ is an ethyl.

**4.** Compound according to any of claims 1 to 3, wherein X is CH.

**5.** Compound according to any of claims 1 to 4 of formula (VI)

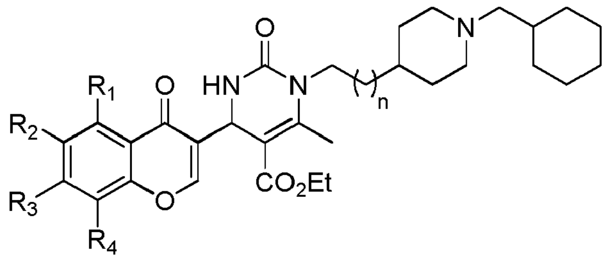

(VI)

**6.** Compound according to any of claims 1 to 4 of formula (VII)

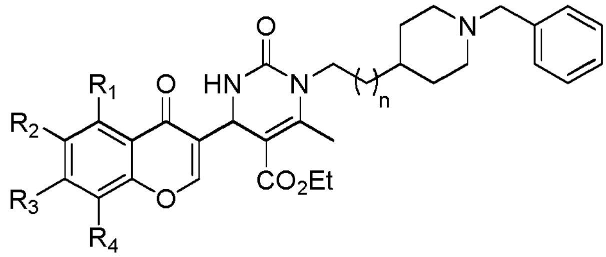

(VII)

**7.** Compound according to any of claims 1 to 6, wherein $R_1$ is H, $R_2$ is a halogen such as Cl, $R_3$ is H or a $C_1$-$C_6$ alkyl such as a methyl, and $R_4$ is H or a halogen such as Cl.

**8.** Compound according to any of claims 1 to 6, wherein $R_1$ and $R_4$ are H, $R_2$ is H or a halogen such as Br, and $R_3$ is H or a $C_1$-$C_6$ alkyl such as an ethyl.

**9.** Compound according to any of claims 1 to 6, wherein $R_1$, $R_3$ and $R_4$ are H, and $R_2$ is a $C_1$-$C_6$ alkyl, in particular an ethyl or an isopropyl.

**10.** Compound according to any of claims 1 to 4 and 6, wherein said compound is chosen in the group consisting of:

BIGI 3h

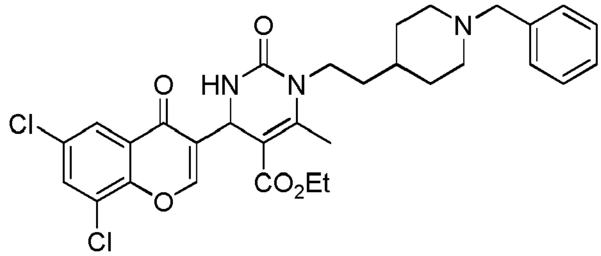

BIGI 3a

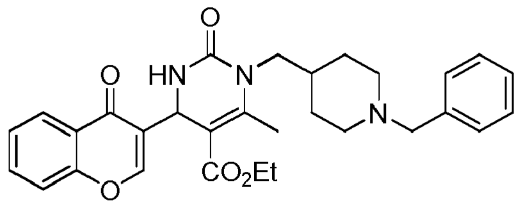

BIGI 3b

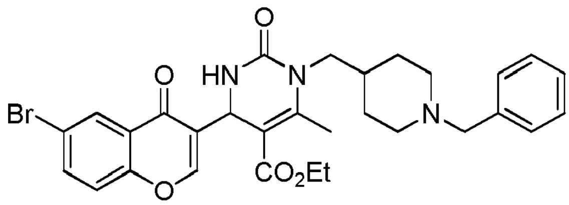

BIGI 3c

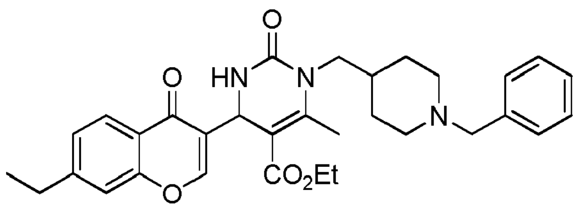

BIGI 3d

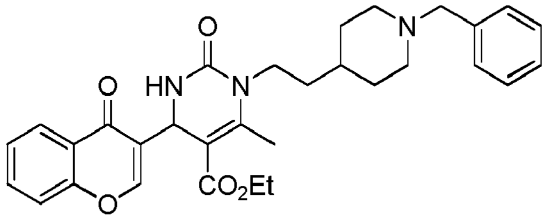

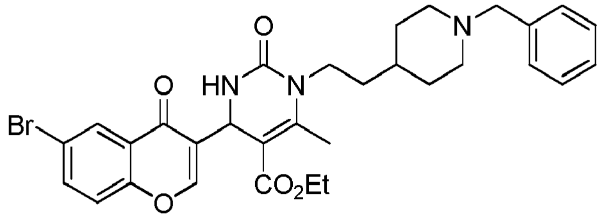

BIGI 3e

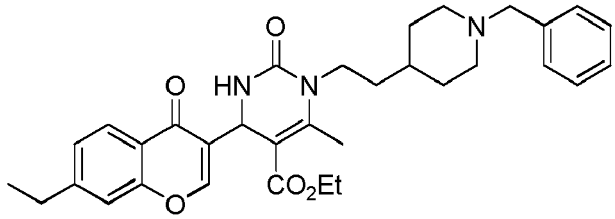

BIGI 3f

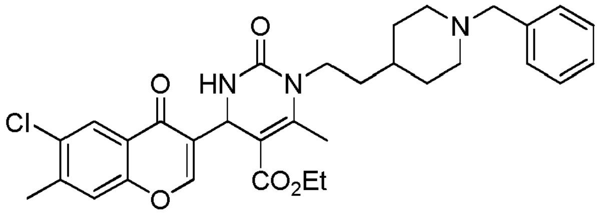

BIGI 3g

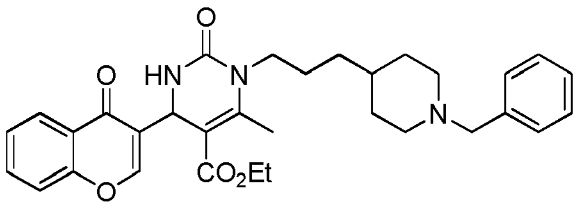

BIGI 3i

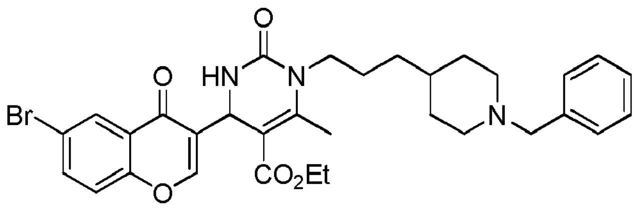

BIGI 3j

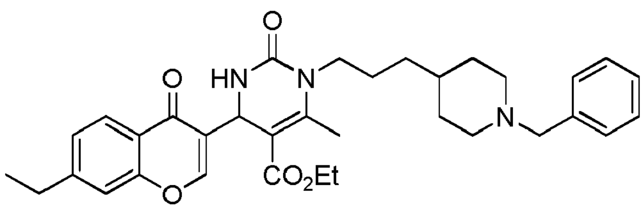

BIGI 3k

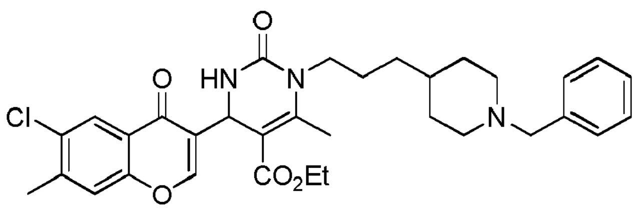

BIGI 3l

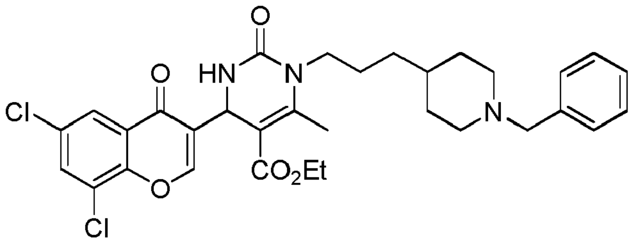

BIGI 3m

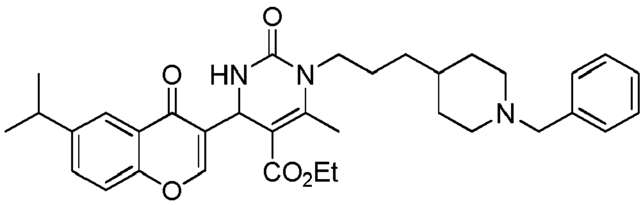

BIGI 3n

**11.** Compound according to any of claims 1 to 6, of formula BIGI 3h

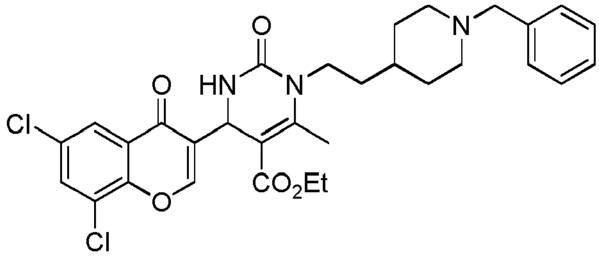

BIGI 3h

**12.** Compound according to any of claims 1 to 3, wherein X is N or a compound according to claims 1 to 3 wherein X is N and of formula (III):

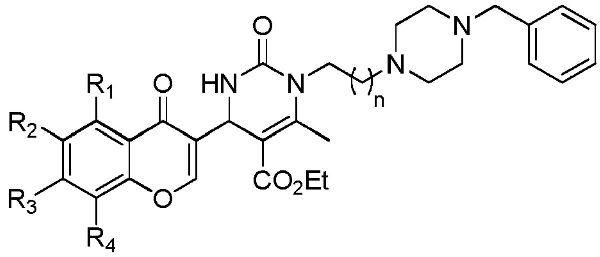

(III)

**13.** A pharmaceutical composition comprising a compound according to any of claims 1 to 12, or a pharmaceutically acceptable salt thereof, and a pharmaceutically acceptable carrier.

**14.** A compound according to any of claims 1 to 12 or a pharmaceutical composition according to claim 13 for use as a medicament.

**15.** A compound according to any of claims 1 to 12 or a pharmaceutical composition according to claim 13, for use in the treatment of a neurodegenerative disease, a central nervous system disease, narcolepsy, or sleep disorder, in particular said neurodegenerative disease is selected in the group consisting of: Alzheimer's disease, Parkinson's disease, amyotrophic lateral sclerosis.

## FIGURE 1

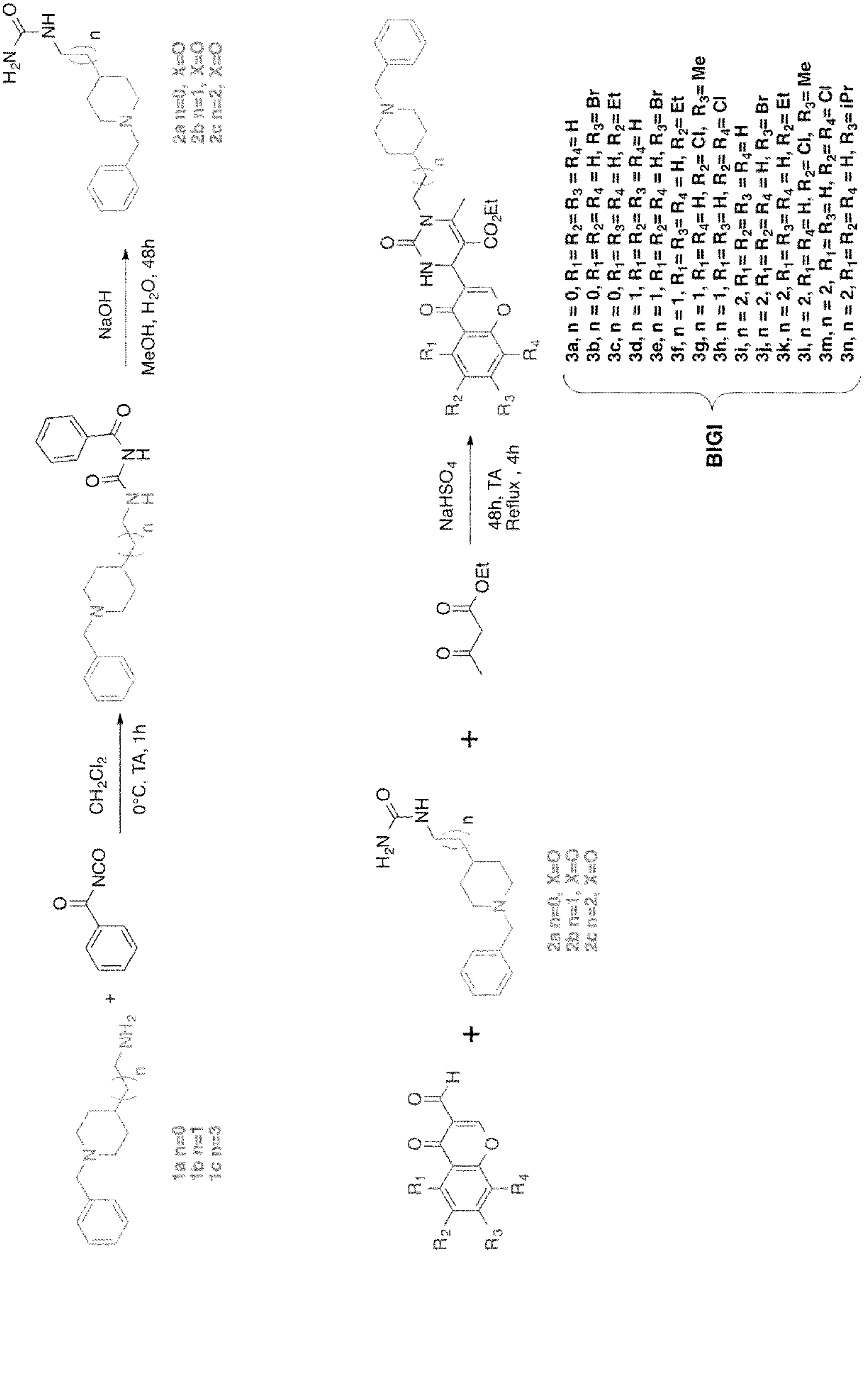


1a n=0
1b n=1
1c n=3

2a n=0, X=O
2b n=1, X=O
2c n=2, X=O

BIGI

3a, n = 0, $R_1 = R_2 = R_3 = R_4 = H$
3b, n = 0, $R_1 = R_2 = R_4 = H$, $R_3 = Br$
3c, n = 0, $R_1 = R_3 = R_4 = H$, $R_2 = Et$
3d, n = 1, $R_1 = R_2 = R_3 = R_4 = H$
3e, n = 1, $R_1 = R_2 = R_4 = H$, $R_3 = Br$
3f, n = 1, $R_1 = R_3 = R_4 = H$, $R_2 = Et$
3g, n = 1, $R_1 = R_4 = H$, $R_2 = Cl$, $R_3 = Me$
3h, n = 1, $R_1 = R_3 = H$, $R_2 = R_4 = Cl$
3i, n = 2, $R_1 = R_2 = R_3 = R_4 = H$
3j, n = 2, $R_1 = R_2 = R_4 = H$, $R_3 = Br$
3k, n = 2, $R_1 = R_3 = R_4 = H$, $R_2 = Et$
3l, n = 2, $R_1 = R_4 = H$, $R_2 = Cl$, $R_3 = Me$
3m, n = 2, $R_1 = R_3 = H$, $R_2 = R_4 = Cl$
3n, n = 2, $R_1 = R_2 = R_4 = H$, $R_3 = iPr$

FIGURE 2

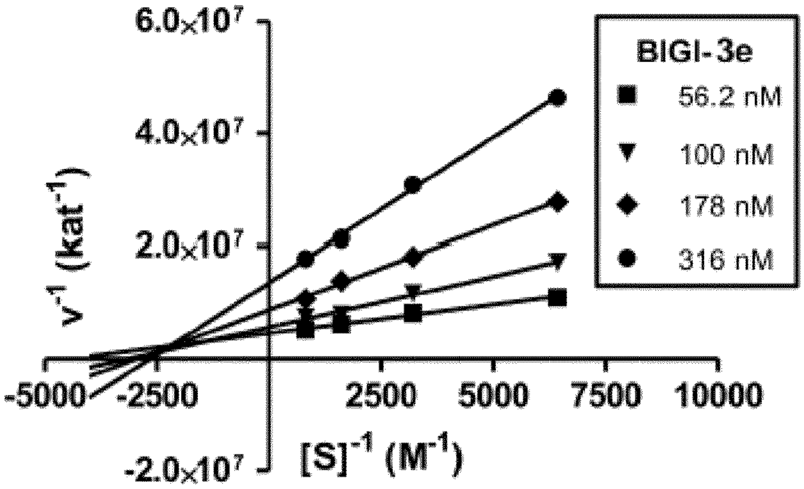


FIGURE 3

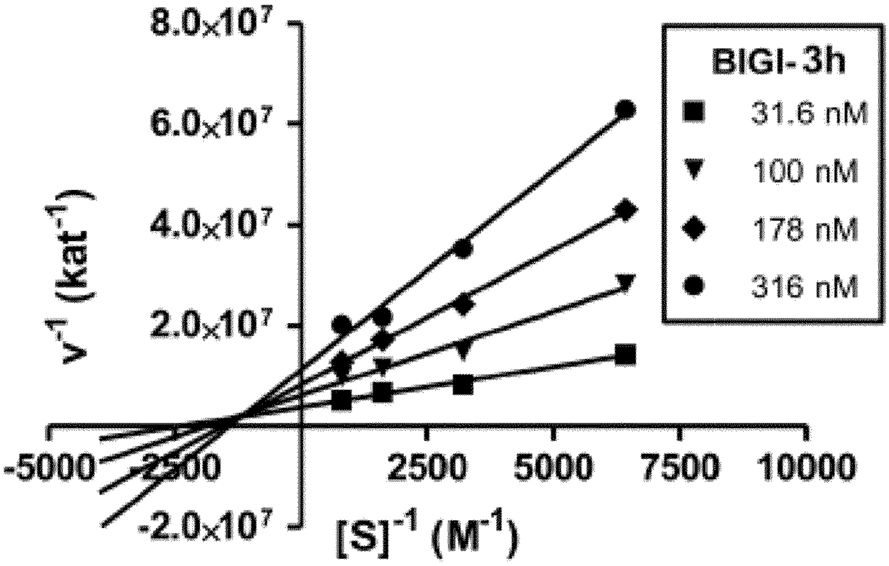

FIGURE 4

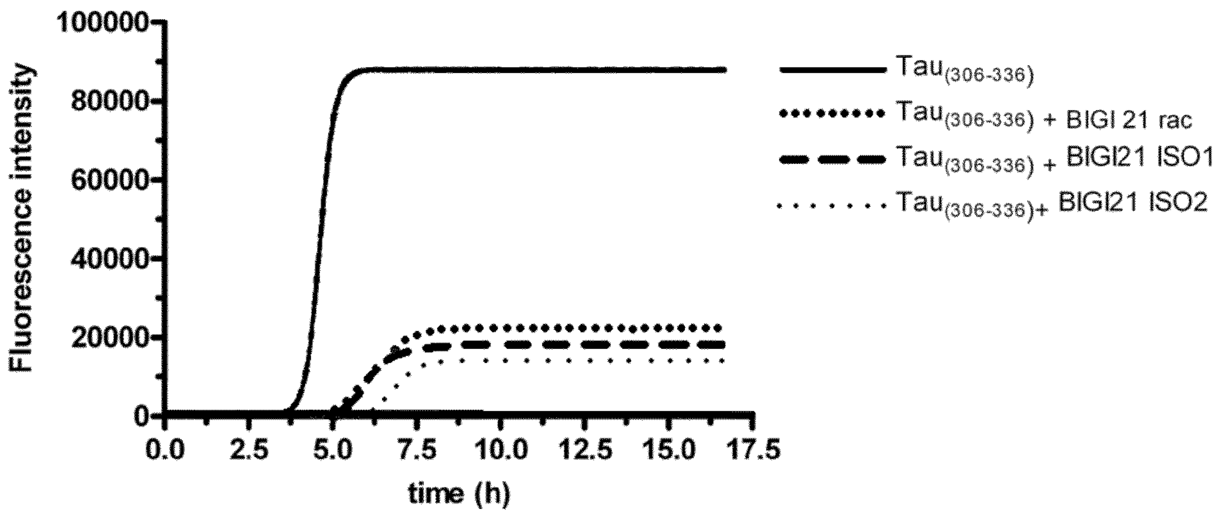


FIGURE 5

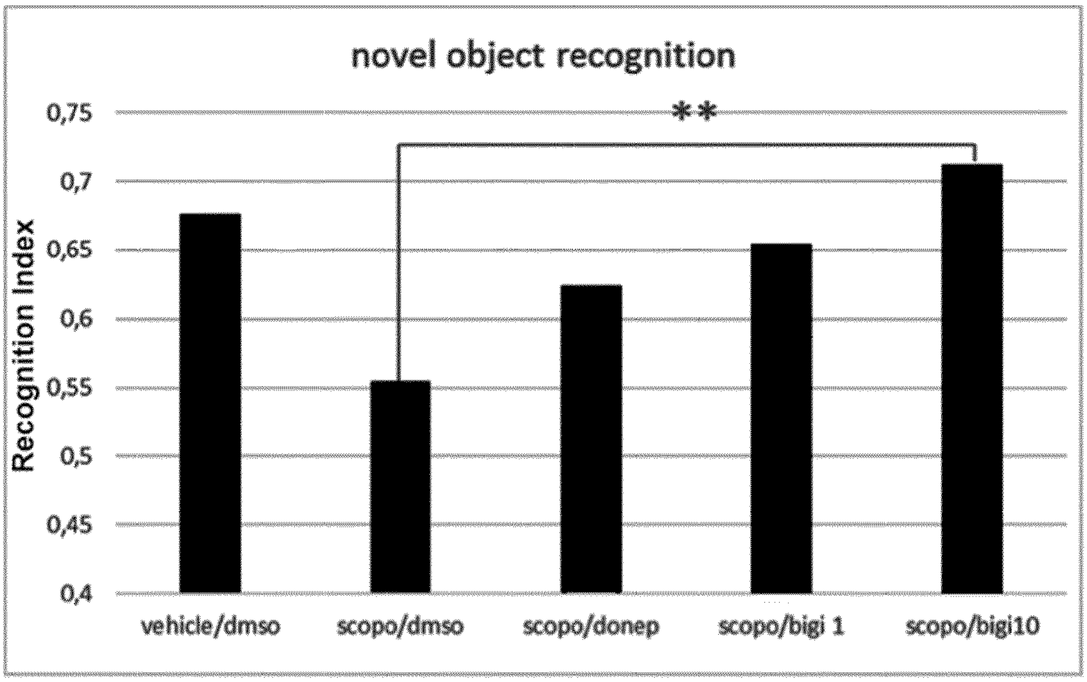

Europäisches Patentamt
European Patent Office
Office européen des brevets

# EUROPEAN SEARCH REPORT

Application Number
EP 20 30 5691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JUSTYNA GODYN ET AL: "Therapeutic strategies for Alzheimer's disease in clinical trials", PHARMACOLOGICAL REPORTS, vol. 68, no. 1, 1 February 2016 (2016-02-01), pages 127-138, XP055471351, PL ISSN: 1734-1140, DOI: 10.1016/j.pharep.2015.07.006 * the whole document * ----- | 1-15 | INV. C07D405/14 A61P25/16 A61P25/28 A61K31/513 |
| A | CHOI DONG-YOUNG ET AL: "Natural products from marine organisms with neuroprotective activity in the experimental models of Alzheimer's disease, Parkinson's disease and ischemic brain stroke: their molecular targets and action me", ARCHIVES OF PHARMACAL RESEARCH, NATL. FISHERIES UNIVERSITY, PUSAN, KR, vol. 38, no. 2, 28 October 2014 (2014-10-28), pages 139-170, XP035442603, ISSN: 0253-6269, DOI: 10.1007/S12272-014-0503-5 [retrieved on 2014-10-28] * the whole document * ----- | 1-15 | |

| TECHNICAL FIELDS SEARCHED (IPC) |
|---|
| A61P C07D |

The present search report has been drawn up for all claims

1

| Place of search | Date of completion of the search | Examiner |
|---|---|---|
| Munich | 22 October 2020 | Seelmann, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5698573 A


### Non-patent literature cited in the description


- **QUERFURTH, H. W. ; LAFERLA, F. M.** Alzheimer's Disease. *N. Engl. J. Med.,* 2010, vol. 362 (4), 329-344
- **BARTUS, R. T. ; DEAN, R. L. ; BEER, B. ; LIPPA, A. S.** The Cholinergic Hypothesis of Geriatric Memory Dysfunction. *Science,* 1982, vol. 217 (4558), 408-414
- **LIU, Z. ; ZHANG, A. ; SUN, H. ; HAN, Y. ; KONG, L. ; WANG, X.** Two Decades of New Drug Discovery and Development for Alzheimer's Disease. *RSC Adv.,* 2017, vol. 7 (10), 6046-6058
- **KUMAR, A. ; SINGH, A. ; EKAVALI, NULL.** A Review on Alzheimer's Disease Pathophysiology and Its Management: An Update. *Pharmacol. Rep. PR,* 2015, vol. 67 (2), 195-203
- **GODYŃ, J. ; JOŃCZYK, J. ; PANEK, D. ; MALAWSKA, B.** Therapeutic Strategies for Alzheimer's Disease in Clinical Trials. *Pharmacol. Rep.,* 2016, vol. 68 (1), 127-138
- **LI, C. ; GÖTZ, J.** Tau-Based Therapies in Neurodegeneration: Opportunities and Challenges. *Nat. Rev. Drug Discov.,* 2017, vol. 16 (12), 863-883
- **AVILA, J. ; PÉREZ, M. ; LIM, F. ; GÓMEZ-RAMOS, A. ; HERNÁNDEZ, F. ; LUCAS, J. J. TAU.** Neurodegenerative Diseases: Tau Phosphorylation and Assembly. *Neurotox. Res.,* 2004, vol. 6 (6), 477-482
- **VILLARROYA, M. ; GARCIA, A. G. ; MARCO-CONTELLES, J. ; LÓPEZ, M. G.** An Update on the Pharmacology of Galantamine. *Expert Opin. Investig. Drugs,* 2007, vol. 16 (12), 1987-1998
- **CHEN, C.-H. ; ZHOU, W. ; LIU, S. ; DENG, Y. ; CAI, F. ; TONE, M. ; TONE, Y. ; TONG, Y. ; SONG, W.** Increased NF-KB Signalling up-Regulates BACE1 Expression and Its Therapeutic Potential in Alzheimer's Disease. *Int. J. Neuropsychopharmacol,* 2012, vol. 15 (1), 77-90
- **CHO, H. J. ; JIN, S. M. ; YOUN, H. D. ; HUH, K. ; MOOK-JUNG, I.** Disrupted Intracellular Calcium Regulates BACE1 Gene Expression via Nuclear Factor of Activated T Cells 1 (NFAT 1) Signaling. *Aging Cell,* vol. 7 (2), 137-147
- **HAYLEY, M. ; PERSPICACE, S. ; SCHULTHESS, T. ; SEELIG, J.** Calcium Enhances the Proteolytic Activity of BACE1: An in Vitro Biophysical and Biochemical Characterization of the BACE1-Calcium Interaction. *Biochim. Biophys. Acta,* 2009, vol. 1788 (9), 1933-1938
- **OULÈS, B. ; DEL PRETE, D. ; GRECO, B. ; ZHANG, X. ; LAURITZEN, I. ; SEVALLE, J. ; MORENO, S. ; PATERLINI-BRÉCHOT, P. ; TREBAK, M. ; CHECLER, F. et al.** Ryanodine Receptor Blockade Reduces Amyloid-β Load and Memory Impairments in Tg2576 Mouse Model of Alzheimer Disease. *J. Neurosci. Off. J. Soc. Neurosci.,* 2012, vol. 32 (34), 11820-11834
- **SMALL, D. H. ; GASPERINI, R. ; VINCENT, A. J. ; HUNG, A. C. ; FOA, L.** The Role of Aβ-Induced Calcium Dysregulation in the Pathogenesis of Alzheimer's Disease. *J. Alzheimers Dis.,* 2009, vol. 16 (2), 225-233
- **CHAKROBORTY, S. ; STUTZMANN, G. E.** Calcium Channelopathies and Alzheimer's Disease: Insight into Therapeutic Success and Failures. *Eur. J. Pharmacol.,* 2014, vol. 739, 83-95
- **BERNHARDI, R. ; EUGENÍN, J.** Alzheimer's Disease: Redox Dysregulation As a Common Denominator for Diverse Pathogenic Mechanisms. *Antioxid. Redox Signal,* 2011, vol. 16 (9), 974-1031
- **PRATICÒ, D.** Oxidative Stress Hypothesis in Alzheimer's Disease: A Reappraisal. *Trends Pharmacol. Sci.,* 2008, vol. 29 (12), 609-615
- **PRATICÒ, D. ; SUNG, S.** Lipid Peroxidation and Oxidative Imbalance: Early Functional Events in Alzheimer's Disease. *J. Alzheimers Dis.,* 2004, vol. 6 (2), 171-175
- **FEDERICO, A. ; CARDAIOLI, E. ; DA POZZO, P. ; FORMICHI, P. ; GALLUS, G. N. ; RADI, E.** Mitochondria, Oxidative Stress and Neurodegeneration. *J. Neurol. Sci.,* 2012, vol. 322 (1-2), 254-262
- **YAN, M. H. ; WANG, X. ; ZHU, X.** Mitochondrial Defects and Oxidative Stress in Alzheimer Disease and Parkinson Disease. *Free Radic. Biol. Med.,* 2013, vol. 62, 90-101

- **GREENOUGH, M. A. ; CAMAKARIS, J. ; BUSH, A. I.** Metal Dyshomeostasis and Oxidative Stress in Alzheimer's Disease. *Neurochem. Int.,* 2013, vol. 62 (5), 540-555
- **CANDORE, G. ; BULATI, M. ; CARUSO, C. ; CASTIGLIA, L. ; COLONNA-ROMANO, G. ; DI BONA, D. ; DURO, G. ; LIO, D. ; MATRANGA, D. ; PELLICANO, M. et al.** Inflammation, Cytokines, Immune Response, Apolipoprotein E, Cholesterol, and Oxidative Stress in Alzheimer Disease: Therapeutic Implications. *Rejuvenation Res.,* 2010, vol. 13 (2-3), 301-313
- **LEE, Y.-J. ; HAN, S. B. ; NAM, S.-Y. ; OH, K.-W. ; HONG, J. T.** Inflammation and Alzheimer's Disease. *Arch. Pharm. Res.,* 2010, vol. 33 (10), 1539-1556
- **MARIN, D. B. ; BIERER, L. M. ; LAWLOR, B. A. ; RYAN, T. M. ; JACOBSON, R. ; SCHMEIDLER, J. ; MOHS, R. C. ; DAVIS, K. L.** L-Deprenyl and Physostigmine for the Treatment of Alzheimer's Disease. *Psychiatry Res.,* 1995, vol. 58 (3), 181-189
- **ROSINI, M. ; SIMONI, E. ; MILELLI, A. ; MINARINI, A. ; MELCHIORRE, C.** Oxidative Stress in Alzheimer's Disease: Are We Connecting the Dots?. *J. Med. Chem.,* 2014, vol. 57 (7), 2821-2831
- **GONZALEZ-LAFUENTE, L. ; EGEA, J. ; LEÓN, R. ; MARTINEZ-SANZ, F. J. ; MONJAS, L. ; PEREZ, C. ; MERINO, C. ; GARCIA-DE DIEGO, A. M. ; RODRIGUEZ-FRANCO, M. I. ; GARCIA, A. G. et al.** Benzothiazepine CGP37157 and Its Isosteric 2'-Methyl Analogue Provide Neuroprotection and Block Cell Calcium Entry. *ACS Chem. Neurosci.,* 2012, vol. 3 (7), 519-529
- **ELLMAN, G. L. ; COURTNEY, K. D. ; ANDRES JR., V. ; FEATHERSTONE, R. M.** A New and Rapid Colorimetric Determination of Acetylcholinesterase Activity. *Biochem. Pharmacol.,* 1961, vol. 7 (2), 88-95
- **NEPOVIMOVA, E. ; KORABECNY, J. ; DOLEZAL, R. ; BABKOVA, K. ; ONDREJICEK, A. ; JUN, D. ; SEPSOVA, V. ; HOROVA, A. ; HRABINOVA, M. ; SOUKUP, O. et al.** Tacrine-Trolox Hybrids: A Novel Class of Centrally Active, Nonhepatotoxic Multi-Target-Directed Ligands Exerting Anticholinesterase and Antioxidant Activities with Low In Vivo Toxicity. *J. Med. Chem.,* 2015, vol. 58 (22), 8985-9003
- **OU, B. ; HAMPSCH-WOODILL, M. ; PRIOR, R. L.** Development and Validation of an Improved Oxygen Radical Absorbance Capacity Assay Using Fluorescein as the Fluorescent Probe. *J. Agric. Food Chem.,* 2001, vol. 49 (10), 4619-4626
- **DÁVALOS, A. ; GÓMEZ-CORDOVÉS, C. ; BARTOLOMÉ, B.** Extending Applicability of the Oxygen Radical Absorbance Capacity (ORAC-Fluorescein) Assay. *J. Agric. Food Chem.,* 2004, vol. 52 (1), 48-54
- **BARTOLINI, M. ; BERTUCCI, C. ; BOLOGNESI, M. L. ; CAVALLI, A. ; MELCHIORRE, C. ; ANDRISANO, V.** Insight into the Kinetic of Amyloid Beta (1-42) Peptide Self-Aggregation: Elucidation of Inhibitors' Mechanism of Action. *Chembiochem Eur. J. Chem. Biol.,* 2007, vol. 8 (17), 2152-2161
- **NAIKI, H. ; HIGUCHI, K. ; HOSOKAWA, M. ; TAKEDA, T.** Fluorometric Determination of Amyloid Fibrils in Vitro Using the Fluorescent Dye, Thioflavin T1. *Anal. Biochem.,* 1989, vol. 177 (2), 244-249
- **LEVINE, H.** 3rd. Thioflavine T Interaction with Synthetic Alzheimer's Disease Beta-Amyloid Peptides: Detection of Amyloid Aggregation in Solution. *Protein Sci. Publ. Protein Soc.,* 1993, vol. 2 (3), 404-410
- **LEMES, L. F. N. ; DE ANDRADE RAMOS, G. ; DE OLIVEIRA, A. S. ; DA SILVA, F. M. R. ; DE CASTRO COUTO, G. ; DA SILVA BONI, M. ; GUIMARÃES, M. J. R. ; SOUZA, I. N. O. ; BARTOLINI, M. ; ANDRISANO, V. et al.** Cardanol-Derived AChE Inhibitors: Towards the Development of Dual Binding Derivatives for Alzheimer's Disease. *Eur. J. Med. Chem.,* 2016, vol. 108, 687-700
- **DI, L. ; KERNS, E. H. ; FAN, K. ; MCCONNELL, O. J. ; CARTER, G. T.** High Throughput Artificial Membrane Permeability Assay for Blood-Brain Barrier. *Eur. J. Med. Chem.,* 2003, vol. 38 (3), 223-232
- **SUGANO, K. ; HAMADA, H. ; MACHIDA, M. ; USHIO, H.** High Throughput Prediction of Oral Absorption: Improvement of the Composition of the Lipid Solution Used in Parallel Artificial Membrane Permeation Assay. *J. Biomol. Screen.,* 2001, vol. 6 (3), 189-196
- **WOHNSLAND, F. ; FALLER, B.** High-Throughput Permeability PH Profile and High-Throughput Alkane/Water Log P with Artificial Membranes. *J. Med. Chem.,* 2001, vol. 44 (6), 923-930
- **BAKI, A. ; BIELIK, A. ; MOLNÁR, L. ; SZENDREI, G. ; KESERÜ, G. M.** A High Throughput Luminescent Assay for Glycogen Synthase Kinase-3beta Inhibitors. *Assay Drug Dev. Technol.,* 2007, vol. 5 (1), 75-83
- **COGHLAN, M. P. ; CULBERT, A. A. ; CROSS, D. A. ; CORCORAN, S. L. ; YATES, J. W. ; PEARCE, N. J. ; RAUSCH, O. L. ; MURPHY, G. J. ; CARTER, P. S. ; ROXBEE COX, L. et al.** Selective Small Molecule Inhibitors of Glycogen Synthase Kinase-3 Modulate Glycogen Metabolism and Gene Transcription. *Chem. Biol.,* 2000, vol. 7 (10), 793-803
- **GANDINI, A. ; BARTOLINI, M. ; TEDESCO, D. ; MARTINEZ-GONZALEZ, L. ; ROCA, C. ; CAMPILLO, N. E. ; ZALDIVAR-DIEZ, J. ; PEREZ, C. ; ZUCCHERI, G. ; MITI, A. et al.** Tau-Centric Multitarget Approach for Alzheimer's Disease: Development of First-in-Class Dual Glycogen Synthase Kinase 3β and Tau-Aggregation Inhibitors. *J. Med. Chem.,* 2018, vol. 61 (17), 7640-7656

- **CHOI, J. Y. ; CALVET, C. M. ; GUNATILLEKE, S. S. ; RUIZ, C. ; CAMERON, M. D. ; MCKERROW, J. H. ; PODUST, L. M. ; ROUSH, W. R.** Rational Development of 4-Aminopyridyl-Based Inhibitors Targeting Trypanosoma Cruzi CYP51 as Anti-Chagas Agents. *J. Med. Chem.,* 2013, vol. 56 (19), 7651-7668
- **KUMAR, B. ; GUPTA, V. P. ; KUMAR, V.** A Perspective on Monoamine Oxidase Enzyme as Drug Target: Challenges and Opportunities. *Curr. Drug Targets,* 2017, vol. 18 (1), 87-97
- **KNEZ, D. ; SOVA, M. ; KOŠAK, U. ; GOBEC, S.** Dual Inhibitors of Cholinesterases and Monoamine Oxidases for Alzheimer's disease. *Future Med. Chem.,* 2017, vol. 9 (8), 811-832
- **REIS, J. ; CAGIDE, F. ; VALENCIA, M. E. ; TEIXEIRA, J. ; BAGETTA, D. ; PEREZ, C. ; URIARTE, E. ; OLIVEIRA, P. J. ; ORTUSO, F. ; ALCARO, S. et al.** Multi-Target-Directed Ligands for Alzheimer's Disease: Discovery of Chromone-Based Monoamine Oxidase/Cholinesterase Inhibitors. *Eur. J. Med. Chem.,* 2018, vol. 158, 781-800
- **COHEN, P. ; GOEDERT, M.** GSK3 Inhibitors: Development and Therapeutic Potential. *Nat. Rev. Drug Discov.,* 2004, vol. 3 (6), 479-487
- **PRATI, F. ; DE SIMONE, A. ; BISIGNANO, P. ; ARMIROTTI, A. ; SUMMA, M. ; PIZZIRANI, D. ; SCARPELLI, R. ; PEREZ, D. I. ; ANDRISANO, V. ; PEREZ-CASTILLO, A. et al.** Multitarget Drug Discovery for Alzheimer's Disease: Triazinones as BACE-1 and GSK-3β Inhibitors. *Angew. Chem. Int. Ed.,* 2015, vol. 54 (5), 1578-1582
- **AVRAHAMI, L. ; LICHT-MURAVA, A. ; EISENSTEIN, M. ; ELDAR-FINKELMAN, H.** GSK-3 Inhibition: Achieving Moderate Efficacy with High Selectivity. *Biochim. Biophys. Acta BBA - Proteins Proteomics,* 2013, vol. 1834 (7), 1410-1414
- **ANTUNES, M. ; BIALA, G.** The Novel Object Recognition Memory: Neurobiology, Test Procedure, and Its Modifications. *Cogn. Process.,* 2012, vol. 13 (2), 93-110